# EUROPEAN PATENT APPLICATION

(11) **EP 2 471 556 A1**
(43) Date of publication of application: **04.07.2012**
(21) Application number: 10821738.1
(22) Date of filing: 05.10.2010
(51) Int. Cl.: A61K 49/00

(54) **CONTRAST AGENT FOR PHOTOACOUSTIC IMAGING AND PHOTOACOUSTIC IMAGING METHOD UTILIZING SAME**

(30) Priority: 05.10.2009 JP 2009231994; 24.06.2010 JP 2010144322
(71) Applicant: Canon Kabushiki Kaisha, Tokyo 146-8501 (JP)
(72) Inventor: YAMAUCHI, Fumio, Tokyo 146-8501 (JP); OGAWA, Satoshi, Tokyo 146-8501 (JP); KANAZAKI, Kengo, Tokyo 146-8501 (JP); INOUE, Sachiko, Tokyo 146-8501 (JP); FUKUI, Tatsuki, Tokyo 146-8501 (JP); MINAMI, Masato, Tokyo 146-8501 (JP); KISHI, Mayuko, Tokyo 146-8501 (JP)
(74) Representative: Weser, Wolfgang
(86) International application number: PCT/JP2010/005958
(87) International publication number: WO 2011/043061

(57) **Abstract**

It is intended to provide a novel contrast agent for photoacoustic imaging that is highly capable of binding to a target molecule and generates high photoacoustic signals. The present invention provides a contrast agent for photoacoustic imaging represented by Formula 1:

MNP-((L)₁-(P)ₘ)ₙ (Formula 1)

(wherein MNP represents a particle containing an iron oxide particle; L represents a linker molecule; P represents a ligand molecule; 1 represents 0 or 1; and m and n represent an integer of 1 or larger), the contrast agent for photoacoustic imaging including: a particle containing an iron oxide particle that absorbs light in a near-infrared region; and at least one or more ligand molecule(s) immobilized on the particle containing an iron oxide particle, wherein the immobilization density of the ligand molecule is equal to or higher than the cell surface density of a target molecule.

## Description

### Technical Field

The present invention relates to a contrast agent for photoacoustic imaging and a photoacoustic imaging method using the same.

### Background Art

Photoacoustic imaging as an optical diagnostic imaging method has been reported in recent years. This method uses the ultrasonic wave's property of less scattering *in vivo* (in samples), compared with light, and images the *in-vivo* density distribution of absorbed light energy in high resolution. Specifically, the method involves: irradiating a living body with pulsed light; detecting signals of acoustic wave (ultrasonic wave) formed from biological tissues that have absorbed the energy of the pulsed light propagated/diffused *in vivo*; and analyzing these signals to obtain the *in-vivo* density distribution of the absorbed light energy. The photoacoustic imaging is a convenient and noninvasive diagnostic method. However, due to light scattering and absorption in biological tissues, the energy of the incident light largely attenuates *in vivo* in a manner dependent on the distance from the body surface. As a result, information about the deep region of the living body is difficult to obtain. The achievement of photoacoustic imaging using near-infrared light has been expected from the viewpoint of tissue penetration.

A contrast agent for photoacoustic imaging for improving detection sensitivity or contrasts in photoacoustic imaging has been reported (Non Patent Literature 1). The contrast agent administered into a living body is distributed in biological tissues to be observed. The contrast agent can then generate acoustic wave by absorbing the energy of pulsed light with which the tissues have been irradiated. Thus, the contrast agent for photoacoustic imaging can increase the apparent absorption coefficient of tissues containing this contrast agent, i.e., can add acoustic wave derived from the contrast agent for photoacoustic imaging to acoustic wave derived from the endogenous tissues. This improves the detection sensitivity of the tissues to be observed. Non Patent Literature 1 discloses a particle containing an iron oxide particle, as an example of a compound serving as the contrast agent for photoacoustic imaging. In this literature, photoacoustic signals have been obtained from a particle containing an iron oxide particle mainly composed of maghemite (γ-Fe₂O₃), demonstrating the practicability of the contrast agent for photoacoustic imaging.

Moreover, a contrast agent for photoacoustic imaging of molecules specifically expressed in lesion areas has been reported (e.g., Patent Literature 1 and Non Patent Literature 2). The contrast agent, which has a ligand molecule that can specifically bind to a particular molecule, binds to the target molecule. Information about the presence or absence and amount of the target molecule and positional information thereof can be obtained by detecting photoacoustic signals from the contrast agent.

Patent Literature 1 and Non Patent Literature 2 disclose a peptide or an antibody labeled with a near-infrared fluorescent dye, as a photoacoustic signal-transmitting part in the contrast agent for photoacoustic imaging. Use of this fluorescent dye-labeled peptide or antibody is considered to achieve photoacoustic imaging, because this peptide or antibody enables the contrast agent to specifically bind to the target molecule. However, this photoacoustic imaging has the problem that the contrast agent for photoacoustic imaging is low sensitive, i.e., the absorption coefficient per molecule of the contrast agent is low. This problem becomes more serious when the expression level of the target molecule is low or when the target molecule is present in the deep region of the living body. On the other hand, a single-chain antibody alone has lower binding performance than that of the whole antibody alone. Moreover, a large number of organic dyes may be bound to the whole antibody. The resulting antibody has insufficient antigen binding performance. Thus, a contrast agent for photoacoustic imaging that has sufficient antigen binding performance even in a dye-bound form has been demanded.

Moreover, Patent Literature 2 discloses a particle containing an iron oxide particle with a single-chain antibody fragment of an anti-CD227 (MUC1) antibody immobilized thereon. However, the particle of Patent Literature 2 is intended to be used in the thermotherapy of tumor. The optimal molecular design as a contrast agent for imaging and its photoacoustic transmission ability are not disclosed by any means.

An iron oxide particle has been applied to the medical field by using its physical properties. The iron oxide particle has already been put in practical use as a contrast agent for magnetic resonance apparatuses (MRI) by using its super paramagnetism. The validity and biological safety of the iron oxide particle as an MRI contrast agent have been confirmed.

Moreover, photoacoustic imaging has been attempted using Resovist (registered trademark), a dextran particle containing an iron oxide particle (Non Patent Literature 3). This approach uses the mechanism under which the iron oxide particle absorbs light to generate sound wave.

Furthermore, a ligand molecule that specifically binds to a particular tissue can be immobilized on an iron oxide particle and used as a probe for diagnostic use. Specific receptors are expressed in cancer tissues, unlike normal tissues. Epidermal growth factor receptor 2 (HER2) is a receptor specifically expressed in cancer tissues. It has been reported that cancer imaging can be achieved by: immobilizing an antibody against HER2 on an iron oxide-containing contrast agent; and detecting, by MRI, the contrast agent accumulated to cancer (Patent Literature 2).

Patent Literature 2 discloses a particle in which a single-chain antibody fragment of an anti-CD227 (MUC1) antibody immobilized on an iron oxide nano particle. However, the particle of Patent Literature 2 is intended to be used in the thermotherapy of tumor. The optimal molecular design as a contrast agent for imaging and its photoacoustic transmission ability are not disclosed by any means.

### Citation List

### Patent Literature

PTL 1: National Publication of International Patent Application No. 2003-500367
PTL 2: WO2008/134586

### Non Patent Literature

NPL 1: IEEE Ultrasonics Symposium 393, (2006)
NPL 2: Bioconjugate Chem., 19 (6), 1186-1193 (2008)
NPL 3: Biomedizinische Technik (Biomedical Engineering) 2009, 54: 83-88

### Summary of Invention

### Technical Problem

An iron oxide particle as a contrast agent for photoacoustic imaging described in Non Patent Literature 1 has no ligand molecule and therefore, hardly specifically images a target molecule, in principle.

A fluorescent dye-modified antibody or peptide described in Patent Literature 1 and Non Patent Literature 2 is capable of specifically binding to a target molecule. Nevertheless, sufficient photoacoustic signals might not be obtained due to a loss of incident light energy from fluorescence emission and a low absorption coefficient per molecule of the fluorescent dye. Furthermore, the modification of an antibody or a peptide with many fluorescent dyes reduces the binding capability of the antibody or the peptide to the target molecule, resulting in limited improvement in sensitivity.

Thus, an object of the present invention is to provide a novel contrast agent for photoacoustic imaging that is highly capable of binding to a target molecule and transmits high photoacoustic signals.

Moreover, in Non Patent Literature 3, Resovist (registered trademark), a dextran particle containing an iron oxide particle, is used as a contrast agent for photoacoustic imaging. However, the Resovist used in this literature is a contrast agent that has been developed for the purpose of imaging the liver by MRI. The iron oxide particle has a particle size as small as 1 to 10 nm, which is suitable for MRI imaging. Thus, this contrast agent for photoacoustic imaging has presented the problem that the obtained signal intensity is weak.

Thus, an object of the present invention is to provide a contrast agent for photoacoustic imaging having an iron oxide particle, wherein the iron oxide particle has a particle size between 15 nm and 500 nm inclusive.

### Solution to Problem

The present invention provides a contrast agent for photoacoustic imaging represented by Formula 1:

MNP-((L)₁-(P)ₘ)ₙ (Formula 1)

(wherein MNP represents a particle containing an iron oxide particle; L represents a linker molecule; P represents a ligand molecule; 1 represents 0 or 1; and m and n represent an integer of 1 or larger),
the contrast agent for photoacoustic imaging including: a particle containing an iron oxide particle that absorbs light in a near-infrared region; and at least one or more ligand molecule(s) immobilized on the particle containing an iron oxide particle, wherein the immobilization density of the ligand molecule is equal to or higher than the cell surface density of a target molecule.

The present invention also provides a contrast agent for photoacoustic imaging having an iron oxide particle, wherein the iron oxide particle has an average particle size between 15 nm and 500 nm inclusive.

### Advantageous Effects of Invention

A contrast agent for photoacoustic imaging of the present invention includes a ligand molecule-immobilized particle containing iron oxide that absorbs light in a high near-infrared region. Therefore, the contrast agent for photoacoustic imaging of the present invention can be used as a highly sensitive contrast agent for photoacoustic imaging.

Particularly, use of a HER2-binding molecule as a ligand molecule can allow the contrast agent to be located in large amounts on HER2-expressing cell surface or in the cells or the neighborhoods thereof. As a result, photoacoustic signals from the HER2-expressing cell surface or within the cells or the neighborhoods thereof are stronger than those from other sites.

Moreover, use of a single-chain antibody as an antibody bound to the particle containing iron oxide can provide a contrast agent for photoacoustic imaging having higher antigen binding performance.

The present invention uses an iron oxide particle emitting strong photoacoustic signals and can therefore provide a contrast agent emitting more excellent photoacoustic signals than those of conventional techniques.

### Brief Description of Drawings

[FIG. 1] FIG. 1 is a schematic diagram illustrating a contrast agent for photoacoustic imaging of the present invention including a ligand molecule and a particle containing an iron oxide particle.
[FIG. 2] FIG. 2 is a graph illustrating the number of antibodies per cell surface HER2 molecule (antibody/HER2 molar ratio) of an antibody-immobilized iron oxide particle as an example of the contrast agent for photoacoustic imaging of the present invention.
[FIG. 3A] FIG. 3A illustrates results of evaluating the HER2 binding capability and binding specificity of an antibody-immobilized iron oxide particle as an example of the contrast agent for photoacoustic imaging of the present invention.
[FIG. 3B] FIG. 3B illustrates results of evaluating the HER2 binding capability and binding specificity of an antibody-immobilized iron oxide particle as an example of the contrast agent for photoacoustic imaging of the present invention.
[FIG. 4A] FIG. 4A illustrates results of measuring the photoacoustic signals of an antibody-immobilized iron oxide particle.
[FIG. 4B] FIG. 4B illustrates results of measuring the photoacoustic signals of an antibody-immobilized iron oxide particle.
[FIG. 4C] FIG. 4C illustrates results of measuring the photoacoustic signals of an antibody-immobilized iron oxide particle.
[FIG. 5] FIG. 5 illustrates results of the pharmacokinetics (at 24 hours after administration) of a ligand molecule-immobilized iron oxide particle administered to cancer-bearing mice.
[FIG. 6] FIG. 6 illustrates results of the pharmacokinetics (at 24 hours after administration) of scFv-immobilized iron oxide particles with varying particle sizes administered to cancer-bearing mice.
[FIG. 7A] FIG. 7A is (1) a graph illustrating the relationship between photoacoustic signals and molar absorption coefficients of particles and is (2) a graph illustrating the relationship between molar absorption coefficients of particles and iron contents in a particle (the amount of iron per particle).
[FIG. 7B] FIG. 7B is (1) a graph illustrating the relationship between photoacoustic signals and molar absorption coefficients of particles and is (2) a graph illustrating the relationship between molar absorption coefficients of particles and iron contents in a particle (the amount of iron per particle).
[FIG. 8] FIG. 8 is a graph illustrating the tumor accumulations (at 24 hours after administration; indicated in %ID/g) of scFv-immobilized iron oxide particles with varying particle sizes administered to cancer-bearing mice.
[FIG. 9] FIG. 9 illustrates results of the pharmacokinetics (at 24 hours after administration) of a HER2-binding peptide-immobilized particle HBP-EO-NP-200 containing an iron oxide particle administered to cancer-bearing mice.
[FIG. 10] FIG. 10 illustrates results of the pharmacokinetics (at 24 hours after administration) of scFv-EO2k-NP-200 and scFv-E05k-NP-200 administered to cancer-bearing mice.
[FIG. 11] FIG. 11 is a simplified diagram of a manufactured photoacoustic imaging apparatus for small animals.
[FIG. 12] FIG. 12 illustrates results of photoacoustic imaging of scFv-EO-NP-200 under the skins of mice.
[FIG. 13A] FIG. 13A is a schematic diagram illustrating the structure of a contrast agent for photoacoustic imaging according to the present embodiment.
[FIG. 13B] FIG. 13B is a schematic diagram illustrating the structure of a contrast agent for photoacoustic imaging according to the present embodiment.
[FIG. 13C] FIG. 13C is a schematic diagram illustrating the structure of a contrast agent for photoacoustic imaging according to the present embodiment.
[FIG. 13D] FIG. 13D is a schematic diagram illustrating the structure of a contrast agent for photoacoustic imaging according to the present embodiment.
[FIG. 13E] FIG. 13E is a schematic diagram illustrating the structure of a contrast agent for photoacoustic imaging according to the present embodiment.
[FIG. 13F] FIG. 13F is a schematic diagram illustrating the structure of a contrast agent for photoacoustic imaging according to the present embodiment.
[FIG. 13G] FIG. 13G is a schematic diagram illustrating the structure of a contrast agent for photoacoustic imaging according to the present embodiment.
[FIG. 13H] FIG. 13H is a schematic diagram illustrating the structure of a contrast agent for photoacoustic imaging according to the present embodiment.
[FIG. 13I] FIG. 13I is a schematic diagram illustrating the structure of a contrast agent for photoacoustic imaging according to the present embodiment.
[FIG. 13J] FIG. 13J is a schematic diagram illustrating the structure of a contrast agent for photoacoustic imaging according to the present embodiment.
[FIG. 14A] FIG. 14A is a diagram illustrating one example of the process of producing a contrast agent for photoacoustic imaging according to the present embodiment.
[FIG. 14B] FIG. 14B is a diagram illustrating one example of the process of producing a contrast agent for photoacoustic imaging according to the present embodiment.
[FIG. 15] FIG. 15 illustrates the waveform of photoacoustic signal intensity.
[FIG. 16A] FIG. 16A illustrates molar absorption coefficients relative to the particle size of an iron oxide particle.
[FIG. 16B] FIG. 16B illustrates molar absorption coefficients relative to the particle size of an iron oxide particle.
[FIG. 16C] FIG. 16C illustrates photoacoustic signals relative to the particle size of an iron oxide particle.
[FIG. 16D] FIG. 16D illustrates photoacoustic signals relative to the particle size of an iron oxide particle.
[FIG. 17A] FIG. 17A illustrates results of evaluating a contrast agent 1 (NP1) for photoacoustic imaging.
[FIG. 17B] FIG. 17B illustrates results of evaluating a contrast agent 1 (NP1) for photoacoustic imaging.
[FIG. 18A] FIG. 18A illustrates results of evaluating a contrast agent 2 (NP2) for photoacoustic imaging.
[FIG. 18B] FIG. 18B illustrates results of evaluating a contrast agent 2 (NP2) for photoacoustic imaging.
[FIG. 19] FIG. 19 is a cryo-TEM image of a contrast agent 3 (NP3) for photoacoustic imaging.
[FIG. 20A] FIG. 20A is a diagram illustrating the relationship between the particle size of a contrast agent for photoacoustic imaging and photoacoustic signals.
[FIG. 20B] FIG. 20B is a diagram illustrating the relationship between the particle size of a contrast agent for photoacoustic imaging and photoacoustic signals.

### Description of Embodiments

Hereinafter, the embodiments of the present invention will be described with reference to the drawings, etc. These embodiments individually disclosed are examples of a contrast agent for photoacoustic imaging of the present invention and photoacoustic imaging using the contrast agent. The technical scope of the present invention is not limited to them.

### (First embodiment)

A contrast agent for photoacoustic imaging according to the first embodiment is represented by Formula 1 and includes: a particle containing an iron oxide particle that absorbs light in a near-infrared region; and at least one or more ligand molecule(s) immobilized on the particle containing an iron oxide particle, wherein the immobilization density of the ligand molecule is equal to or higher than the cell surface density of a target molecule.

MNP-((L)₁-(P)ₘ)ₙ (Formula 1)

(wherein MNP represents a particle containing an iron oxide particle; L represents a linker molecule; P represents a ligand molecule; l represents 0 or 1; and m and n represent an integer of 1 or larger).

The contrast agent for photoacoustic imaging of the present embodiment has been achieved focusing on the high absorption coefficient, in a near-infrared region, of the particle containing an iron oxide particle. The feature of this contrast agent is that: the particle containing an iron oxide particle that absorbs light in a near-infrared region can be used in combination with the ligand molecule in photoacoustic imaging; and the immobilization density of the ligand molecule is equal to or higher than the cell surface density of a target molecule.

The present invention is the first case showing that: a complex including a particle containing an iron oxide particle and a targeting molecule is suitable as a contrast agent for photoacoustic imaging; and the targeting molecule having an immobilization density equal to or higher than the cell surface density of a target molecule is suitable for a contrast agent for photoacoustic imaging.

### (Contrast agent for photoacoustic imaging)

In the present invention, the "contrast agent" is defined as a compound that can enhance the contrast between tissues or molecules to be observed mainly in samples and their neighboring tissues or molecules and improve the detection sensitivity of morphological or positional information of the tissues or molecules to be observed. In this context, the "contrast agent for photoacoustic imaging" means a contrast agent used in photoacoustic imaging.

### (Iron oxide particle)

The iron oxide particle used in the present embodiment is not particularly limited as long as the iron oxide particle absorbs light in a near-infrared wavelength region between 600 nm and 1300 nm inclusive to transmit photoacoustic signals and is harmless to human bodies. One of Fe₃O₄ (magnetite), γ-Fe₂O₃ (maghemite), and a mixture thereof can be used. Particularly, magnetite can be used. The magnetite is known to have a higher molar absorption coefficient in a near-infrared wavelength region than that of maghemite and thus considered to emit stronger photoacoustic signals. Use of the magnetite as a signal-transmitting part can improve sensitivity, compared with conventional contrast agents for photoacoustic imaging. Moreover, the iron oxide particle may be in a crystalline state selected from the group consisting of single crystal, polycrystal, and amorphous. Moreover, the number of the iron oxide particle may be one. Alternatively, a secondary particle including two or more iron oxide particles may be used. The iron oxide particle may have a large particle size, for example, 50 nm. In such a case, the value of a molar absorption coefficient per mol of iron atoms and the value of a photoacoustic signal measured per mol of iron atoms are increased with increases in the number of the iron oxide particle contained in the contrast agent. On the other hand, the iron oxide particle may have a particle size of 5 nm. In such a case, the values of a molar absorption coefficient and a measured photoacoustic signal per mol of iron atoms do not much vary even when the number of the iron oxide particle contained in the contrast agent is increased.

The iron oxide particle that can be used in the present embodiment has a particle size of 1 to 1000 nm, particularly 1 to 100 nm. The particle size of the iron oxide particle can be measured by a method known in the art such as transmission electron microscope (TEM) observation or X-ray diffraction. The iron oxide particle can be prepared appropriately for use by an iron oxide particle preparation method known in the art. Alternatively, a commercially available product may be used.

In this context, the near-infrared light or the light in a near-infrared region refers to electromagnetic wave having a wavelength of 600 to 2500 nm in a near-infrared region. In the present embodiment, light in a near-infrared region having a wavelength of 600 to 1300 nm can be used. Particularly, light in a near-infrared region having a wavelength of 700 to 800 nm can be used. This is because the light in this wavelength region is low absorbed by molecules in samples, can relatively deeply penetrate the tissues of samples, and is safe to samples.

The absorption region of the contrast agent for photoacoustic imaging of the present embodiment can be, but not limited to, 600 to 2500 nm in a near-infrared region. Ultraviolet light and light in a visible region of 700 nm or lower can also be used for in-sample observation or ex-sample observation of the subject to be observed after administration into the samples.

### (Particle containing iron oxide particle)

In the present embodiment, the particle containing an iron oxide particle means a particle consisting only of the iron oxide particle or a particle containing the iron oxide particle in a matrix such as a polymer (e.g., dextran) or silica. The particle containing an iron oxide particle according to the present embodiment can have a diameter of 1 to 1000 nm from a hydrodynamic standpoint. In this context, the "matrix" refers to a compound that is capable of containing therein or retaining the iron oxide particle and forming a complex with the iron oxide particle. The matrix may be any compound as long as the compound can stably contain therein or retain the iron oxide particle. The matrix can be a hydrophilic polymer such as dextran, polyamino acid, polyethylene glycol, a poly(lactic acid-co-glycolic acid), albumin, or collagen from the viewpoint of water solubility or water dispersibility. Among them, particularly, dextran can be used.

The weight ratio between the matrix, for example, dextran, and the iron oxide particle (matrix (g)/iron oxide (g)), which constitute the particle containing an iron oxide particle, can be 1 or more. This value represents the coated state of the iron oxide particle. The smaller the value becomes, the more likely the surface of the iron oxide particle is to be exposed, resulting in the poorer dispersion stability of the particle. This weight ratio can usually be 0.1 to 10, particularly, in the range of 1 to 5.

Moreover, the matrix can be a compound that has the effect of suppressing the aggregation of the particle containing an iron oxide particle and has a functional group for immobilizing a ligand molecule. For example, the compound can be dextran, and the functional group can be selected from the group consisting of carboxyl, amino, maleimide, and hydroxyl groups. The functional group can be introduced to the dextran according to a chemical modification method known in the art. For example, a dextran particle can be cross-linked to epichlorohydrin and then treated with ammonia to prepare a dextran particle having an amino group. The particle containing an iron oxide particle can be prepared appropriately for use by a particle-containing particle preparation method known in the art. Alternatively, a commercially available product may be used.

### (Ligand molecule)

The "ligand molecule" means a compound that can specifically bind to a target molecule. Moreover, the phrase "specifically bind" described here is defined as a dissociation constant KD (lower value means higher binding affinity) of 1 µM or lower for the target molecule. The ligand molecule is not particularly limited as long as the ligand molecule is a compound having a dissociation constant of 1 µM or lower for the target molecule. Examples thereof include enzymes, antibodies, receptors, cytokines, hormones, and serum proteins. The ligand molecule of the present embodiment can be, particularly, an antibody from the viewpoint of a low dissociation constant and molecular structural stability.

The "antibody" described here is a generic name for proteins of the immunoglobulin family induced by the immune system in response to particular antigens or substances. The antibody is a substance that can recognize a particular target molecule and bind to this target molecule. The antibody can be selected from the group consisting of mouse, human, humanized, and chimeric antibodies or may be derived from other species. Moreover, the antibody can be selected from the group consisting of monoclonal and polyclonal antibodies. Furthermore, an antibody fragment, a portion of the antibody, which is a lower-molecular-weight derivative of the antibody capable of binding to a target molecule may be used. Examples of the antibody fragment include a Fab fragment (hereinafter, also abbreviated to "Fab"), a Fab' fragment (hereinafter, also abbreviated to "Fab "'), F(ab'), F(ab')₂, a heavy chain variable (VH) domain alone, a light chain variable (VL) domain alone, a VH-VL complex, a camelized VH domain, and a peptide containing an antibody complementarity determining region (CDR).

Of them, particularly, a single-chain antibody (scFv) having a heavy chain variable region and a light chain variable region linked via a peptide linker can be used. Furthermore, a humanized single-chain antibody can particularly be used. The single-chain antibody can be prepared economically and conveniently according to various antigens and has a smaller molecular weight than that of usual antibodies. Thus, use of the single-chain antibody can increase the amount of the antibody immobilized per the particle containing an iron oxide particle. Furthermore, the single-chain antibody is free from the Fc domain (constant domain) of the antibody and can therefore reduce antigenicity (antigenicity in an individual that receives the contrast agent for photoacoustic imaging of the present invention). Therefore, the single-chain antibody can particularly be used as ligand molecule in the contrast agent for photoacoustic imaging of the present embodiment.

### (Target molecule)

The contrast agent for photoacoustic imaging of the present embodiment can be used, particularly in photoacoustic imaging for the diagnosis of affected tissues such as tumor. Thus, in the present embodiment, the "target molecule" is not particularly limited as long as the target molecule is a molecule derived from sample organisms. The target molecule means a molecule specifically expressed on lesion areas in samples, particularly, a molecule specifically expressed in tumor sites in samples. Examples thereof include tumor antigens, receptors, cell surface membrane proteins, proteases, and cytokines. The target molecule according to the present embodiment can be a tumor antigen.

Specific examples of the tumor antigen include vascular endothelial growth factor (VEGF) family, vascular endothelial growth factor receptor (VEGFR) family, prostate specific antigen (PSA), carcinoembryonic antigen (CEA), matrix metalloproteinase (MMP) family, epidermal growth factor receptor (EGFR) family, epidermal growth factor (EGF), integrin family, type 1 insulin-like growth factor receptor (IGF-1R), CD184 antigen (CXC chemokine receptor 4: CXCR4), and placental growth factor (PIGF). Particularly, epidermal growth factor receptor 2 (HER2) of the EGFR family can be used.

Ligand molecules that specifically bind to the tumor antigen can be obtained easily by those skilled in the art. For example, antibodies can be prepared appropriately for use by an antibody preparation method known in the art with the antigen or a partial peptide thereof as an immunogen. From the gene sequence information of the prepared antibodies, a single-chain antibody can also be obtained as a recombinant protein by a gene recombination method. Alternatively, a commercially available product may be used.

### (Binding reaction between particle containing iron oxide particle and ligand molecule)

The particle containing an iron oxide particle is directly bound to the ligand molecule by conventional well known coupling reaction with a functional group (e.g., a carboxyl, amino, maleimide, or hydroxyl group) present on the surface of the particle containing an iron oxide particle, as a reactive group. Specific examples of the direct binding include amidation reaction. This reaction is performed, for example, by the condensation of a carboxyl group or its ester derivative with an amino group. The carboxyl group can be amidated directly using a carbodiimide condensing agent such as N,N'-dicyclohexylcarbodiimide or 1-ethyl-3-(3-dimethylaminopropyl)carbodiimide hydrochloride. Alternatively, the carboxyl group can also be converted in advance to active ester using N-hydroxysuccinimide (NHS) to promote amidation reaction.

In addition, binding reaction between thiol and maleimide groups can also be used. In this reaction, efficient and selective binding reaction can be performed in a neutral region. The particle containing an iron oxide particle bound to the ligand molecule by the reaction can be washed or purified by ultrafiltration or gel filtration chromatography. Moreover, a ferromagnetic form of the particle containing an iron oxide particle can be washed or purified by magnetic separation using a permanent magnet. On the other hand, a particle containing a very small iron oxide particle exhibiting super paramagnetism can be washed or purified using a magnetic column under a high-gradient magnetic field.

### (Linker molecule)

Moreover, in the contrast agent for photoacoustic imaging of the present embodiment, the binding between the particle containing an iron oxide particle and the ligand molecule is not limited to direct binding and may be appropriate binding via a linker molecule. In this context, the "linker molecule" is defined as a compound that links the particle containing an iron oxide particle to the ligand molecule. The linker molecule that can be used is selected from the group consisting of bifunctional short-chain alkanes (e.g., alkanedithiol and alkanediamine) and bifunctional polyethylene oxide. For example, a terminal group of the linker molecule may be bound to the functional group of the particle containing an iron oxide particle, while the other terminal group of the linker molecule may be bound to the functional group of the ligand molecule.

In the Formula 1, the number of the linker molecule represented by I is 0 or I. When 1 is 0, the particle containing an iron oxide particle is directly bound to the ligand molecule. In this case, m is 1 and is equal to n, and the number of the ligand molecule immobilized on the particle is n. On the other hand, when 1 is 1, the particle containing an iron oxide particle is bound to the ligand molecule via a linker molecule L. When both I and m are 1, the number of the ligand molecule immobilized on the particle is n. However, the linker molecule may be a polyfunctional molecule. In such a case, a plurality of ligand molecules is bound per linker molecule. Specifically, when I is 1 and m is 2 or larger, the number of the ligand molecule immobilized on the particle is mxn. Particularly, I and m can be 1.

The method for binding the particle containing an iron oxide particle and the ligand molecule to the linker molecule and the type of the linker molecule are not particularly limited to those described above and can be selected appropriately by those skilled in the art from various available binding methods and linker molecules known in the art. The contrast agent for photoacoustic imaging of the present invention can be intended to be dispersed stably in water from the viewpoint of in-sample use. In this regard, a water-soluble linker molecule can particularly be used. For example, bifunctional polyethylene oxide can be used. As described later in Examples, use of polyethylene oxide as a linker molecule not only improves the yield of the particle of the contrast agent (ratio of the amount of final purification products to the amount of feed) but also improves the dispersion stability of the contrast agent for photoacoustic imaging in water. The molecular weight of the polyethylene oxide can be selected arbitrarily without impairing the functions of the particle containing an iron oxide particle and the ligand molecule. Excessive increase in the molecular weight of the polyethylene oxide would reduce the efficiency of binding reaction of the ligand molecule to the surface of the particle containing an iron oxide particle. Thus, polyethylene oxide having an average molecular weight of 100 to 2000, particularly, an average molecular weight of 300, can be used. Thus, its average molecular weight can be 100 to 10000, particularly, in the range of 100 to 5000. Polyethylene oxide having an average molecular weight of 300 is more suitable.

### (Particle size of contrast agent for photoacoustic imaging)

The average particle size of the contrast agent for photoacoustic imaging of the present embodiment is 1 to 500 nm and can be 10 to 200 nm, from a hydrodynamic standpoint. Increase in the particle size of the contrast agent would enhance the phagocytosis of the contrast agent, reduce its half-life in blood, and reduce tissue diffusion. The contrast agent may be used by appropriately adjusting the optimal particle size according to the location site of the target molecule. For example, the particle size of the contrast agent may be as large as approximately 1 µm for target molecules present in blood vessels. On the other hand, the hydrodynamic average particle size of the contrast agent can be 1 to 500 nm, particularly, 10 to 200 nm, for extravascular target molecules. From the viewpoint of photoacoustic signal intensity, a high iron content per particle is appropriate. Therefore, a particle size of 100 to 500 nm can be used from the viewpoint of both pharmacokinetics and photoacoustic signals. As described later in Examples, an iron oxide particle that has a high iron content and has a 171.7-nm (number-average distribution) targeting molecule was confirmed to have a high absorption coefficient, high binding capability to the target molecule, and favorable tumor accumulation, demonstrating its high practicability as a contrast agent for photoacoustic imaging.

In the transport of the contrast agent to general extravascular tumor tissues, the contrast agent having a small particle size is probably advantageous, from the viewpoint of the tissue diffusion of the contrast agent, to migration from within blood vessels to extravascular tissues and a migration pathway to the target molecule in the extravascular tissues. On the other hand, the particle containing an iron oxide particle has a larger average particle size due to the ligand molecule immobilized on the surface. Therefore, a small-size ligand molecule can be suitable for the photoacoustic imaging of tumor in extravascular tissues. Thus, a single-chain antibody having a smaller molecular weight than that of usual antibodies can particularly be used as a ligand molecule in the contrast agent for photoacoustic imaging of the present embodiment. Moreover, the average particle size of the contrast agent for photoacoustic imaging can be measured by a method known in the art such as dynamic light scattering.

### (Functional affinity of contrast agent for photoacoustic imaging)

The contrast agent for photoacoustic imaging of the present embodiment has at least one or more ligand molecule(s) per the particle containing an iron oxide particle. The number (valence) of the ligand molecule bound per the particle containing an iron oxide particle, i.e., the immobilization density (ng/cm²) of the ligand molecule, would largely influence the binding capability of the contrast agent for photoacoustic imaging to a target molecule. Specifically, multivalent binding effect caused by the increase of the valence of the ligand molecule potentiates the functional affinity (also called "apparent affinity"; hereinafter, also referred to as "binding capability") of the contrast agent for photoacoustic imaging. Owing to the potentiated functional affinity, the contrast agent for photoacoustic imaging can more strongly bind to the target molecule, achieving high-contrast photoacoustic imaging. Moreover, the functional affinity can be evaluated by a method such as ELISA (enzyme-linked immunosorbent assay), spectroscopic methods, quartz-crystal microbalance (QCM), or surface plasmon resonance (SPR) phenomena. The dissociation constant (or "apparent dissociation constant") between the contrast agent for photoacoustic imaging and the target molecule should be 1 µM or lower and can be 10 nM or lower, particularly, 1 nM or lower.

In the Formula 1, the number of the ligand molecule immobilized on the particle is represented by mxn. As described later, larger integers represented by m and n are suitable for enhancing the functional affinity of the contrast agent. For the present invention, it is essential to immobilize the ligand molecule at an immobilization density equal to or higher than the cell surface density of a target molecule. Thus, n is a value depending on the cell surface density of a target molecule. In general, m can be 1, and n can be selected from 1 to 300000. Particularly, m can be 1, and n can be in the range of 2 to 50, for an iron oxide particle having a particle size of approximately 20 nm. A larger particle size enables immobilization of a larger number of ligand molecules. For example, a few hundreds to several tens of thousands of ligand molecules can be immobilized on a 200-nm iron oxide particle. The number of the ligand molecule immobilized depends on the size of the ligand molecule.

Moreover, the immobilization density of the ligand molecule would influence not only the binding capability of the contrast agent for photoacoustic imaging to the target molecule but also the dispersion stability or pharmacokinetics thereof. For example, the particle containing an iron oxide particle, which has a maleimide group on the surface, may have poor dispersion stability in water due to its hydrophobicity. In such a case, the increase of the immobilization density of the ligand molecule can improve the dispersion stability of the particle containing an iron oxide particle.

### (Improvement in binding capability of contrast agent for photoacoustic imaging)

The immobilization density of the ligand molecule equal to or higher than the density of the target molecule present on cell surface (cell surface density) can probably achieve a high binding state. Thus, if the cell surface density of the target molecule is determined, the contrast agent for photoacoustic imaging having the corresponding desired immobilization density of the ligand molecule can be designed to effectively cause multivalent binding effect. In this regard, important is to what extent the ligand molecule in the immobilized state maintains its binding activity. Depending on this extent, the relationship between the immobilization density and the binding capability can vary. Moreover, the number of the targeting molecule that can be immobilized on the surface of the particle containing an iron oxide particle has the upper limit depending on the surface area of the particle containing an iron oxide particle and the number of active groups and further depends on the size of the ligand molecule. Thus, for improving the binding capability of the contrast agent for photoacoustic imaging, important are the rational immobilization of the ligand molecule based on the target molecule density, the size of the ligand molecule, and the binding activity maintenance ratio of the ligand molecule after immobilization.

As described later in Examples, a contrast agent having high binding capability and binding selectivity for HER2 is taken as one example of the contrast agent for photoacoustic imaging of the present embodiment. Specifically, the following structure is required for the immobilization density of the ligand molecule to be equal to or higher than the cell surface density of HER2: as described later in Examples, the targeting molecule is immobilized on the particle containing an iron oxide particle (hydrodynamic diameter: 20 nm) at a proportion of 3 or more, particularly, 7 or more molecules per the particle containing an iron oxide particle. Furthermore, a single-chain antibody was selected, and its immobilization on the particle containing an iron oxide particle used cysteine introduced by mutagenesis to the C-terminus of the single-chain antibody and adopted site-directed immobilization. As a result, the single-chain antibody was immobilized at a large immobilization density, which exceeded the cell surface density of HER2, on the particle containing an iron oxide particle, while the binding activity of the single-chain antibody was maintained. Consequently, the HER2 binding capability of the particle containing an iron oxide particle was successfully improved largely.

### (Examples of contrast agent for photoacoustic imaging that can be used)

In the photoacoustic imaging of the present embodiment, a photoacoustic signal from the contrast agent for photoacoustic imaging administered into samples, i.e., the sound pressure of acoustic wave, is proportional to the absorption coefficient and concentration of the contrast agent for photoacoustic imaging. Thus, properties required for the contrast agent for photoacoustic imaging are a high absorption coefficient and high binding capability to the target molecule.

An example of the contrast agent for photoacoustic imaging that can be used in the present embodiment is a contrast agent for photoacoustic imaging having a HER2-binding single-chain antibody immobilized on a dextran matrix containing magnetite (hydrodynamic diameter: 20 nm). As described later in Examples, the single-chain antibody is immobilized at a proportion of 3 or more, particularly 6 or more molecules per the particle containing an iron oxide particle for achieving high binding capability to HER2. Such a structure can allow the single-chain antibody to have an immobilization density equal to or higher than the cell surface density of HER2.

In consideration of the general cell surface density of HER2 (10⁶ HER2 molecules/cell and 1 pmol HER2/cm² in terms of 10 µm cell diameter) in HER2-positive cells (strongly expressing strain), the contrast agent for photoacoustic imaging can have sufficient multivalent binding to HER2 and can probably achieve strong binding capability. Use of a magnetite (iron oxide) particle that transmits high photoacoustic signals has the advantage that the magnetite particle not only contributes to improvement in sensitivity, a bottleneck of photoacoustic imaging, but also is free from strict limitation on dose because of being harmless to samples. The contrast agent for photoacoustic imaging thus rationally designed has not been reported so far, and its practicability has been proved for the first time by the present invention.

### (Photoacoustic imaging method using contrast agent for photoacoustic imaging)

The contrast agent for photoacoustic imaging of the present embodiment can be used in the photoacoustic imaging of the target molecule. Specifically, a photoacoustic imaging method for a target molecule of the present embodiment includes at least: administering the contrast agent for photoacoustic imaging of the present embodiment to a sample or a sample obtained from the sample; irradiating the sample or the sample obtained from the sample with pulsed light; and measuring a photoacoustic signal derived from the contrast agent for photoacoustic imaging bound to the target molecule present in the sample or in the sample obtained from the sample.

One example of the target molecule imaging method of the present embodiment is as follows: the contrast agent for photoacoustic imaging of the present embodiment is administered to a sample or added to a sample obtained from the sample, such as an organ. In this context, the sample refers to every organism including, but not particularly limited to, mammals such as humans, experimental animals, and pets, and other organisms. Examples of the sample in the sample can include organs, tissues, tissue sections, cells, and cell lysates. After the administration or addition of the contrast agent for photoacoustic imaging, the sample or the like is irradiated with laser pulsed light having a wavelength in a near-infrared range.

A photoacoustic signal (acoustic wave) from the contrast agent for photoacoustic imaging is detected using an acoustic wave detector, for example, a piezoelectric transducer, and converted to an electric signal. Based on this electric signal obtained using an acoustic wave detector, the position or size of an absorber in the sample or the like and the distribution of optical characteristic values such as absorption coefficients can be calculated. For example, when the photoacoustic signal is detected at a value equal to or higher than a reference threshold, this sample can be presumed to contain the target molecule or a site producing the target molecule. Alternatively, the sample can be presumed to contain the target molecule or a site producing the target molecule, or the sample from which the sample is derived can be presumed to contain a site producing the target molecule.

### (Usage of contrast agent for photoacoustic imaging)

The contrast agent for photoacoustic imaging of the present embodiment can be used in the photoacoustic imaging of the target molecule, for example, a tumor antigen specifically expressed in tumor, based on the principles described above. For example, the contrast agent for photoacoustic imaging can be used in the diagnosis of tumor having correlation with an antigen level and can also be used, particularly in a photoacoustic imaging method for a tumor antigen related to breast cancer. The contrast agent for photoacoustic imaging can also be used for the purpose of studying disease with cultured cells or tissues as an assay sample. Moreover, the contrast agent for photoacoustic imaging of the present embodiment may be used in the screening of the target molecule.

Moreover, for the purpose of diagnosing the condition of a patient with the disease or preventively diagnosing the disease in a healthy individual, the contrast agent can also be used in the photoacoustic imaging method for the target molecule by introducing the contrast agent to a sample or cells or tissues obtained from the sample. A diagnostic method based on photoacoustic imaging using the contrast agent for photoacoustic imaging of the present embodiment includes: introducing the contrast agent for photoacoustic imaging to cultured cells, or cells or tissues collected from a sample, or the sample; and detecting the disease-related target molecule to monitor the position and severity of the disease.

Examples of particular usages thereof include the photoacoustic imaging of HER2 using the contrast agent for photoacoustic imaging of the present embodiment, specifically, the contrast agent for photoacoustic imaging according to the present embodiment having a HER2-binding molecule as a ligand molecule. The HER2 described here is also called ErbB2, c-Erb-B2, and p185^{HER2}. The HER2 is a tyrosine kinase-type receptor of the EGFR family. The HER2 is a substance (protein) gene-amplified and overexpressed in adenocarcinomas (hereinafter, also abbreviated to "HER2-expressing cells") such as breast cancer, prostatic cancer, gastric cancer, ovarian cancer, and lung cancer. The HER2 is activated by the formation of a dimer of HER2 molecules (also referred to as a homodimer) or a dimer of HER2 with another EGFR molecule (also referred to as a heterodimer). More specifically, the homodimer or heterodimer formation is considered to cause autophosphorylation, which then induces cell growth signal transduction to the nuclei, resulting in cell growth, infiltration, metastasis, apoptosis inhibition, etc.

In this context, HER2 is hardly internalized in the form of a heterodimer formed with another molecule of the EGFR family. Specifically, in this situation, the HER2-mediated cellular uptake of substances hardly occurs, resulting in the reduced uptake of extracellular substances by HER2-expressing cells. Thus, the inhibition of HER2 heterodimer formation probably allows HER2 to easily migrate into HER2-expressing cells.

In the contrast agent for photoacoustic imaging of the present embodiment, the immobilization density equal to or higher than the cell surface density of HER2 is achieved by immobilizing a large number of HER2-binding antibodies on the particle containing an iron oxide particle. Therefore, the contrast agent for photoacoustic imaging of the present embodiment can inhibit heterodimer formation by the promotion of HER2 homodimer formation, by the binding of the contrast agent to HER2 ahead of the binding of another member of the EGFR family to the HER2, or by the direct dissociation of the heterodimer by the contrast agent.

Specifically, the contrast agent for photoacoustic imaging of the present embodiment is located in larger amounts in HER2-expressing cells or the neighborhood thereof than at other sites, because the contrast agent binds to HER2. Furthermore, the binding of the contrast agent to HER2 inhibits heterodimer formation, allowing HER2 to easily remain in the HER2-expressing cells. Therefore, the contrast agent is more likely to be located in the HER2-expressing cells. Owing to such synergistic effect, the concentration of the contrast agent is enhanced in the HER2-expressing cells or the neighborhood thereof to produce strong photoacoustic signals.

### (Second embodiment)

### (Particle containing iron oxide particle with HER2-binding single-chain antibody)

The second embodiment of the present invention includes a compound represented by Formula 2, the compound having: a particle containing an iron oxide particle; and at least one or more epidermal growth factor receptor 2 (HER2)-binding single-chain antibody(ies) (scFv(s)).

MNP-(-(L)₁-(P)ₘ)ₙ (Formula 2)

(wherein MNP represents a particle containing an iron oxide particle; L represents a linker molecule; P represents an epidermal growth factor receptor 2 (HER2)-binding single-chain antibody (scFv); l represents 0 or 1; and m and n represent an integer of 1 or larger).

The compound can be used as the contrast agent for photoacoustic imaging of the present invention and may be used as a contrast agent for magnetic resonance imaging (MRI). Furthermore, the compound having a ferromagnetic or paramagnetic particle containing an iron oxide particle can be used in the magnetic separation or purification of HER2 by using the property of specifically binding to HER2.

### (Third embodiment)

A contrast agent for photoacoustic imaging according to the present embodiment has a particle containing iron oxide, wherein the particle is bound to a single-chain antibody. The present inventor found that a single-chain antibody bound to a particle containing iron oxide has higher binding performance than that of the whole antibody bound to a particle containing iron oxide. This may be because the single-chain antibody can be bound at a site other than the antigen recognition site to the particle containing iron oxide, whereas the whole antibody is bound at its antigen recognition site to the particle containing iron oxide.

### (Fourth embodiment)

A contrast agent for photoacoustic imaging according to the present embodiment has an iron oxide particle, wherein the iron oxide particle has a particle size between 15 nm and 500 nm inclusive. The iron oxide particle having a particle size of 15 nm or larger has larger values of a molar absorption coefficient and a measured photoacoustic signal per mol of iron atoms than those of an iron oxide particle having a particle size smaller than 15 nm. Therefore, large photoacoustic signals can be obtained in a photoacoustic imaging method using the contrast agent for photoacoustic imaging according to the present embodiment. Moreover, the iron oxide particle can particularly have a particle size of 20 nm or larger.

The contrast agent for photoacoustic imaging according to the present embodiment can have a particle size between 15 nm and 1000 nm inclusive. The contrast agent for photoacoustic imaging having a particle size of 1000 nm or smaller can be accumulated in larger amounts at tumor sites than at normal sites *in vivo* owing to EPR (Enhanced Permeability and Retention) effect. As a result, in a living body that has received the contrast agent, photoacoustic signals emitted from tumor sites are larger than those emitted from normal sites, upon irradiation with light. Thus, the tumor sites can be detected specifically using the contrast agent for photoacoustic imaging according to the present embodiment having a particle size adjusted to 1000 nm or smaller. Moreover, the particle size of the contrast agent for photoacoustic imaging can be 500 nm or smaller, particularly, 200 nm or smaller. The contrast agent for photoacoustic imaging having a particle size of 200 nm or smaller can hardly be incorporated by macrophages in blood, resulting in enhanced retention in blood.

The contrast agent for photoacoustic imaging according to the present embodiment may have only one iron oxide particle or may have two or more iron oxide particles.

In the present embodiment, the iron oxide particle has a particle size of 500 nm or smaller. In the present embodiment, the particle size of the iron oxide particle can be, particularly, 200 nm or smaller. Moreover, in the present embodiment, when the iron oxide particle has a particle size of 500 nm, the contrast agent for photoacoustic imaging can have three or less iron oxide particles.

### (Iron oxide particle)

The iron oxide particle used in the present embodiment is not particularly limited as long as the iron oxide particle absorbs light in a near-infrared wavelength region between 600 nm and 1300 nm inclusive to transmit photoacoustic signals and is harmless to human bodies. One of Fe₃O₄ (magnetite), γ-Fe₂O₃ (maghemite), and a mixture thereof can be used. Particularly, magnetite can be used. The magnetite is known to have a higher molar absorption coefficient in a near-infrared wavelength region than that of maghemite and thus considered to emit stronger photoacoustic signals. Use of the magnetite as a signal-transmitting part can improve sensitivity, compared with conventional contrast agents for photoacoustic imaging. Moreover, the iron oxide particle may be in a crystalline state selected from the group consisting of single crystal, polycrystal, and amorphous. Moreover, the number of the iron oxide particle in contrast agents for photoacoustic imaging may be one or may be two or more. Such two or more iron oxide particles constitute a secondary particle. The iron oxide particle may have a large particle size, for example, 50 nm. In such a case, the value of a molar absorption coefficient per mol of iron atoms and the value of a photoacoustic signal measured per mol of iron atoms are increased with increases in the number of the iron oxide particle contained in the contrast agent. On the other hand, the iron oxide particle may have a particle size of 5 nm. In such a case, the values of a molar absorption coefficient and a measured photoacoustic signal per mol of iron atoms do not much vary even when the number of the iron oxide particle contained in the contrast agent is increased.

The particle size of the iron oxide particle of the present embodiment can be measured by a method known in the art such as transmission electron microscope (TEM) observation or X-ray diffraction. The iron oxide particle can be prepared appropriately for use by an iron oxide particle preparation method known in the art. Alternatively, a commercially available product may be used.

The iron oxide particle according to the present embodiment or the secondary particle of the iron oxide particles may be surface-coated with a surface modification material. Examples of the surface modification material include fatty acid and an amphiphilic compound. Examples of the fatty acid include oleic acid. Examples of the amphiphilic compound include phospholipid, polyoxyethylene sorbitan fatty acid ester, and an amphiphilic polymer. Moreover, a plurality of iron oxide particles may be contained in a hydrophobic polymer, which is in turn bound to an amphiphilic compound. These surface modification materials may be used alone or in combination of two or more thereof. The iron oxide particle according to the present embodiment may be commercially available or may be obtained for use by the following method: for example, FeCl₃ and FeCl₂ are dissolved in water to prepare a solution, which is then supplemented with ammonia water with stirring to prepare an iron oxide particle having elements Fe and O.

### (Contrast agent for photoacoustic imaging)

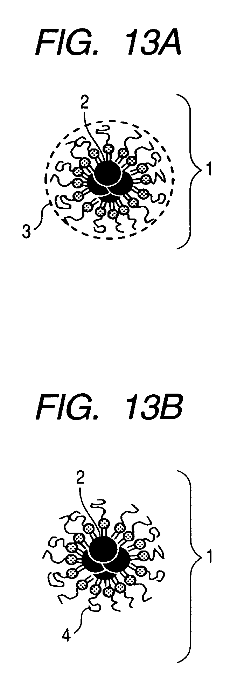

FIGS. 13A to 13J are respectively a schematic diagram illustrating one example of the contrast agent for photoacoustic imaging of the present embodiment. A contrast agent 1 for photoacoustic imaging includes, as illustrated in FIG. 13A, an iron oxide particle 2 and an amphiphilic compound 3. The amphiphilic compound 3 is present on the surface of the contrast agent 1 for photoacoustic imaging.

The amphiphilic compound 3 used may be one or more kinds of amphiphilic compounds selected singly or in combination from the phospholipid 4, the polyoxyethylene sorbitan fatty acid ester 5, or the amphiphilic polymer 6..

The iron oxide particle 2 may be contained in a hydrophobic polymer 7 selected from the group consisting of 1) a homopolymer including a monomer having hydroxycarboxylic acid having 6 or less carbon atoms, or a copolymer including two kinds of monomers having the hydroxycarboxylic acid, 2) poly(styrene), and 3) poly(methyl methacrylate).

FIG. 13B is a schematic diagram illustrating the contrast agent 1 for photoacoustic imaging including the iron oxide particle 2 coated only with the phospholipid 4.

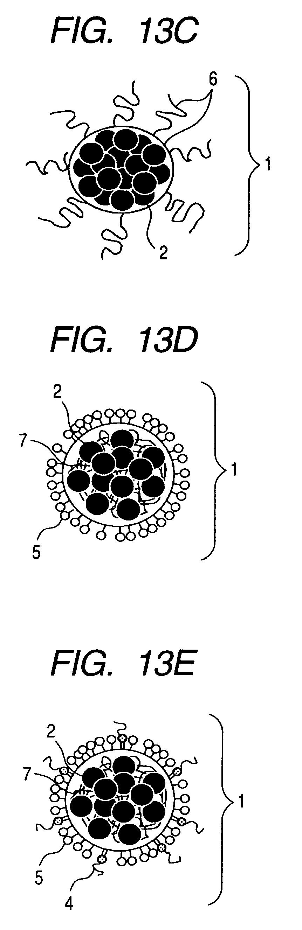

FIG. 13C is a schematic diagram illustrating the contrast agent 1 for photoacoustic imaging including the iron oxide particle 2 coated only with the amphiphilic polymer 6.

FIG. 13D is a schematic diagram illustrating the contrast agent 1 for photoacoustic imaging including the iron oxide particle 2 contained in the hydrophobic polymer 7 and further coated only with the polyoxyethylene sorbitan fatty acid ester 5.

FIG. 13E is a schematic diagram illustrating the contrast agent 1 for photoacoustic imaging including the iron oxide particle 2 contained in the hydrophobic polymer 7 and further coated with both compounds, i.e., the phospholipid 4 and the polyoxyethylene sorbitan fatty acid ester 5.

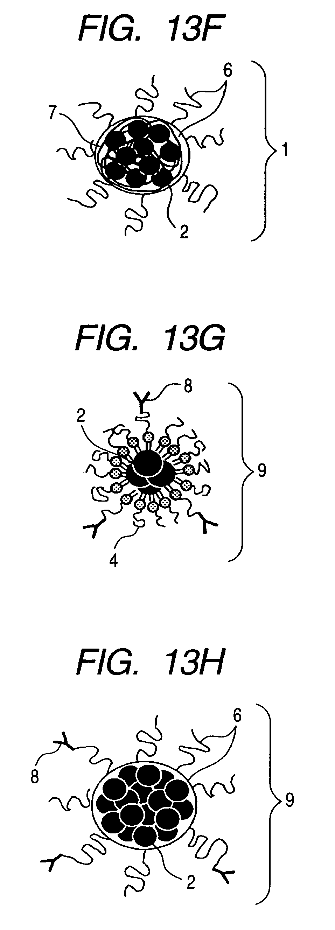

FIG. 13F is a schematic diagram illustrating the contrast agent 1 for photoacoustic imaging including the iron oxide particle 2 contained in the hydrophobic polymer 7 and further coated only with the amphiphilic polymer 6.

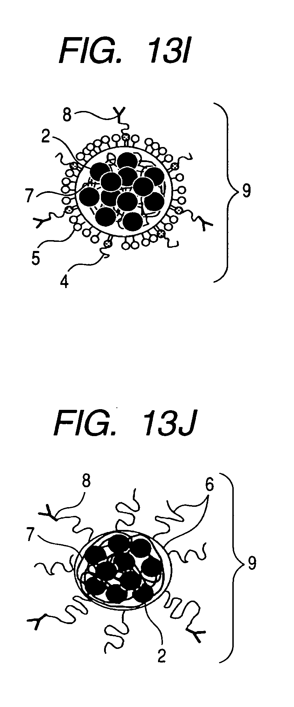

In the contrast agent 1 for photoacoustic imaging having phospholipids 4, a ligand molecule 8 can be immobilized on at least some of the phospholipids 4. FIGS. 13G and 13I are respectively a schematic diagram illustrating a contrast agent 9 for photoacoustic imaging, which is the contrast agent 1 for photoacoustic imaging having the ligand molecule 8-immobilized phospholipid 4. The contrast agent 9 for photoacoustic imaging thus obtained can specifically recognize a target tissue, cell, and substance.

Moreover, in the contrast agent 1 for photoacoustic imaging having amphiphilic polymers 6, the ligand molecule 8 can be immobilized on at least some of the amphiphilic polymers 6. FIGS. 13H and 13J are respectively a schematic diagram illustrating a contrast agent 9 for photoacoustic imaging, which is the contrast agent 1 for photoacoustic imaging having the ligand molecule 8-immobilized amphiphilic polymer 6. The contrast agent 9 for photoacoustic imaging thus obtained can specifically recognize a target tissue, cell, and substance.

Moreover, the particle sizes of the contrast agents 1 and 9 for photoacoustic imaging of the present embodiment can be controlled according to the intended usage. The particle size is between 15 nm and 500 nm inclusive. A contrast agent having a particle size exceeding 200 nm is susceptible to phagocytosis by macrophages, resulting in reduced retention in blood. Therefore, the particle size of the contrast agent for photoacoustic imaging can be controlled to between 15 nm and 200 nm inclusive for imaging blood vessels and accumulating the contrast agent for photoacoustic imaging to cancer.

### (Method for producing contrast agent for photoacoustic imaging)

A method for producing the contrast agent 1 for photoacoustic imaging according to the present embodiment will be described.
Examples of the method for obtaining the contrast agent 1 for photoacoustic imaging of the present embodiment can include, but not limited to, dry up and nanoemulsion methods described below.

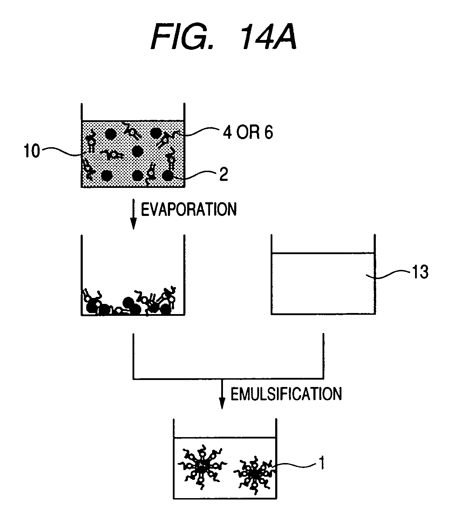

FIG. 14A illustrates one example of the process of producing the contrast agent 1 for photoacoustic imaging by the dry up method. Specifically, a water dispersion of the contrast agent for photoacoustic imaging can be obtained through the following (1) to (2):
(1) evaporating an organic solvent from a first liquid 10 containing the iron oxide particle 2 and the phospholipid 4 or the amphiphilic polymer 6 added to the organic solvent; and
(2) emulsifying the residue after addition of water 13.

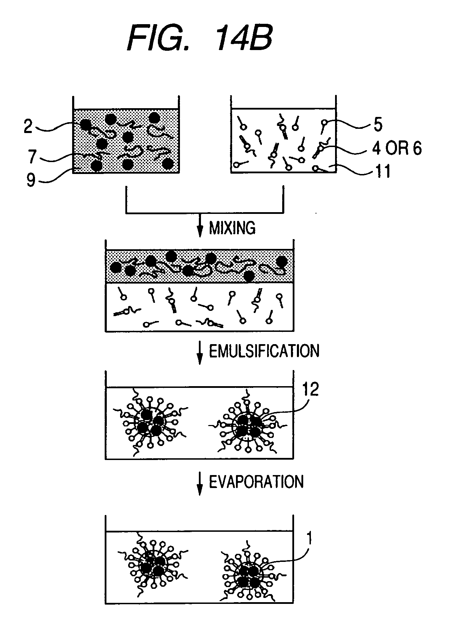

FIG. 14B illustrates one example of the process of producing the contrast agent 1 for photoacoustic imaging by the nanoemulsion method. Specifically, a water dispersion of the contrast agent for photoacoustic imaging can be obtained through the following (1) to (3):
(1) adding a first liquid 10 to a second liquid 11 to obtain a mixed solution, wherein the first liquid 10 contains the iron oxide particle 2 and the hydrophobic polymer 7 added to an organic solvent, and the second liquid 11 contains the phospholipid 4 and the polyoxyethylene sorbitan fatty acid ester 5 or the amphiphilic polymer 6 added to water;
(2) emulsifying the mixed solution to obtain an O/W-type emulsion 12; and
(3) evaporating the organic solvent from the dispersoid of the emulsion 12.

The iron oxide particle may be surface-coated with, for example, oleic acid. An organic solvent is added to the dried iron oxide particle to prepare a dispersion of the iron oxide particle. To this dispersion, oleic acid is added to coat the surface of the iron oxide particle with the oleic acid. Then, oleic acid presented at sites other than the surface of the iron oxide particle can be washed off with an organic solvent to obtain an oleic acid-coated iron oxide particle. The surface modification material is not limited to oleic acid as long as the compound achieves the purpose of enhancing affinity for the amphiphilic compound 3 or dispersing the iron oxide particle 2 in the organic solvent contained in the first liquid 10.

The contrast agent for photoacoustic imaging of the present embodiment may contain one iron oxide particle or may contain two or more iron oxide particles. The present inventor further found that the effect of enhancing a photoacoustic signal per iron atom is obtained by allowing the contrast agent for photoacoustic imaging to contain a plurality of iron oxide particles. Thus, stronger photoacoustic signals can be obtained.

### (Phospholipid)

Some of the phospholipids 4 can include phosphatidyl phospholipid having a functional group selected from the group consisting of amino, carboxyl, NHS (N-hydroxysuccinimide), maleimide, methoxy, and hydroxy groups, and a PEG chain. Phospholipid containing an amino, carboxyl, NHS, or maleimide group as a functional group can immobilize thereon the ligand molecule 8.

Examples thereof can include phospholipids such as N-(aminopropyl polyethyleneglycol)carbonyldistearoylphosphatidyl-ethanolamine (DSPE-PEG-NH₂) represented by the chemical formula 1, 3-(N-glutaryl)aminopropyl, polyethyleneglycol-carbonyl distearoylphosphatidyl-ethanolamine (DSPE-PEG-COOH) represented by the chemical formula 2, 3-(N-succinimidyloxyglutaryl)aminopropyl, polyethyleneglycol-carbonyl distearoylphosphatidyl-ethanolamine (DSPE-PEG-NHS) represented by the chemical formula 3, N-[(3-maleimide-l-oxopropyl)aminopropyl polyethyleneglycol-carbonyl] distearoylphosphatidyl-ethanolamine (DSPE-PEG-MAL) represented by the chemical formula 4, N-(carbonylmethoxypolyethyleneglycol)-1,2-distearoyl-sn-glycero-3-phosphoethanolamine, sodium salt (DSPE-PEG-CN) represented by the chemical formula 5, and 1,2-distearoyl-sn-glycero-3-phosphoethanolamine-N-[poly(ethylene glycol)] (DSPE-PEG) represented by the chemical formula 6.

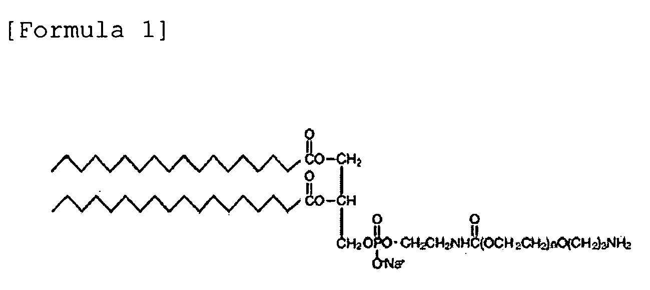

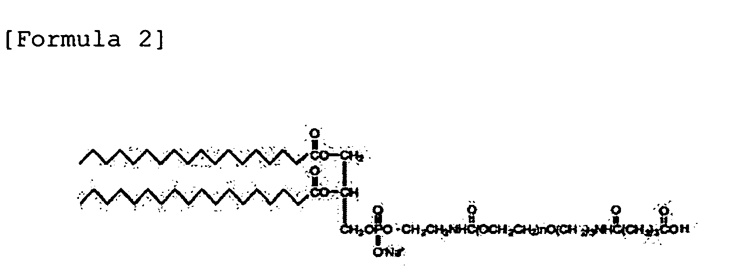

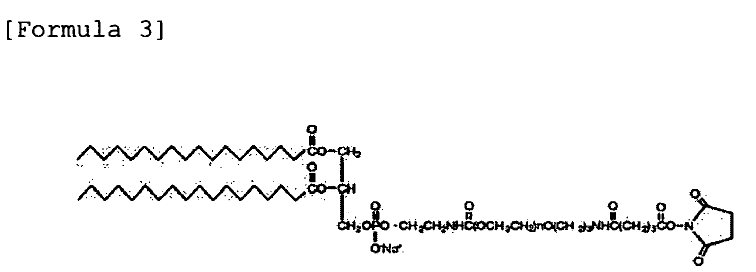

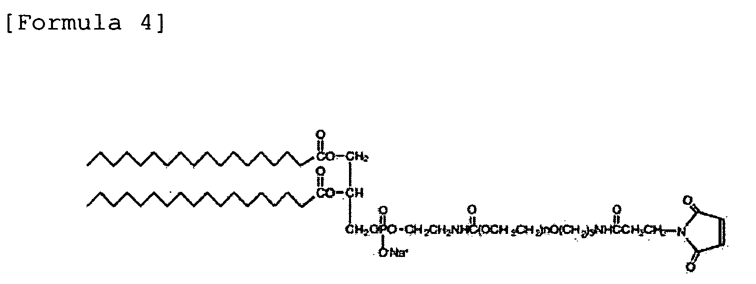

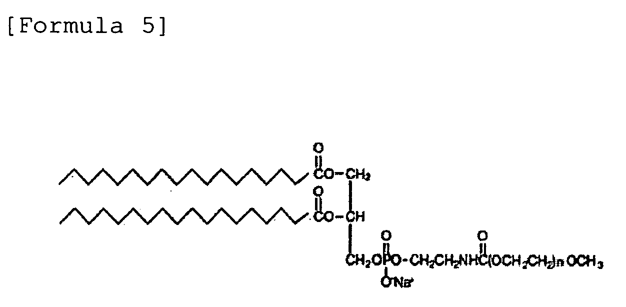

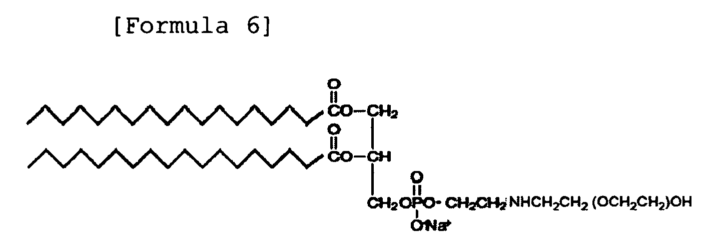

### (Polyoxyethylene sorbitan fatty acid ester)

Examples of the polyoxyethylene sorbitan fatty acid ester 5 according to the present embodiment can include Tween 20 (polyoxyethylene sorbitan monolaurate), Tween 40, Tween 60, and Tween 80.

### (Amphiphilic polymer)

Examples of the amphiphilic polymer 6 according to the present embodiment can include a poly(maleic anhydride-*alt*-octadecen).) having a PEG chain introduced therein, a poly(maleic anhydride-*co*-styrene) having a PEG chain introduced therein, poly(lactic acid) having a PEG chain introduced therein, a poly(lactic acid-*co*-glycolic acid) having a PEG chain introduced therein, poly(ethylene glycol-*co*-propyleneoxide) poly(vinyl alcohol).

The PEG chain that can be used suitably has a weight-average molecular weight of 1000 to 100000, particularly, 2000 to 20000.

Moreover, the PEG chain has a terminal functional group selected from the group consisting of hydroxy, methoxy, amino, carboxyl, NHS, and maleimide groups. A PEG chain containing an amino, carboxyl, NHS, or maleimide group as a functional group can immobilize thereon the ligand molecule 8.

### (Hydrophobic polymer)

The hydrophobic polymer 7 according to the present embodiment is selected from the group consisting of a homopolymer including a monomer having hydroxycarboxylic acid having 6 or less carbon atoms, or a copolymer including two kinds of monomers having the hydroxycarboxylic acid, poly(styrene)
, and poly(methyl methacrylate). These polymers that can be used suitably have a weight-average molecular weight of 2000 to 1000000, particularly, 10000 to 600000.

### (First liquid)

The first liquid 10 used in the dry up method is a solution containing the iron oxide particle 2 and the phospholipid 4 or the amphiphilic polymer 6 dispersed and dissolved in an organic solvent. The first liquid 10 used in the nanoemulsion method is a solution containing the iron oxide particle 2 and the hydrophobic polymer 7 dispersed and dissolved in an organic solvent.

Any solvent is applicable to the organic solvent contained in the first liquid 10 as long as the solvent is an organic solvent that can dissolve the phospholipid 4 or the hydrophobic polymer 7 and disperse the iron oxide particle 2. A volatile organic solvent can be used.

Specific examples of such an organic solvent include the following solvents: halogenated hydrocarbons (dichloromethane, chloroform, chloroethane, dichloroethane, trichloroethane, carbon tetrachloride, etc.), ethers (ethyl ether, isobutyl ether, butanol, etc.), esters (ethyl acetate, butyl acetate, etc.), and aromatic hydrocarbons (benzene, toluene, xylene, etc.). These solvents may be used alone or can also be used as a mixture of two or more thereof at an appropriate ratio. However, the organic solvent contained in the first liquid 10 is not limited to those listed above.

Moreover, the concentration of the phospholipid 4, the amphiphilic polymer 6, or the hydrophobic polymer 7 in the first liquid 10 is not particularly limited within any range that permits their dissolution. The concentration can be set to, for example, 1 to 100 mg/ml for the phospholipid 4, 1 to 100 mg/ml for the amphiphilic polymer 6, and 0.5 to 100 mg/ml for the hydrophobic polymer 7.

Moreover, the weight ratio between the phospholipid 4 and the iron oxide particle 2 contained in the first liquid 10 in the dry up method can range from 10:1 to 1:10. The weight ratio between the amphiphilic polymer 6 and the iron oxide particle 2 contained in the first liquid 10 in the dry up method can range from 10:1 to 1:100. The weight ratio between the hydrophobic polymer 7 and the iron oxide particle 2 contained in the first liquid 10 in the nanoemulsion method can range from 1000:1 to 1:9.

### (Second liquid)

The second liquid 11 used in the nanoemulsion method is an aqueous solution containing one or more kinds of amphiphilic compounds selected singly or in combination from the phospholipid 4, the polyoxyethylene sorbitan fatty acid ester 5, or the amphiphilic polymer 6 for the purpose of stabilizing an emulsion in mixing with the first liquid 10. ( However, the present invention is not limited to this method as long as the dispersion of the first liquid 10 mixed with water can contain the phospholipid 4, the polyoxyethylene sorbitan fatty acid ester 5, or the amphiphilic polymer 6.

Moreover, the concentrations of the phospholipid 4 and the polyoxyethylene sorbitan fatty acid ester 5 contained in the second liquid 11 in the nanoemulsion method differ depending on a mixing ratio to the first liquid 10. For example, the concentration of the phospholipid 4 can be set to 0.001 mg/ml to 100 mg/ml. Moreover, the concentration of the polyoxyethylene sorbitan fatty acid ester 5 can be set to 0.1 mg/ml to 100 mg/ml. The concentration of the amphiphilic polymer 6 can be set to 1 mg/ml to 100 mg/ml.

### (Emulsification)

In the dry up method, a mixed solution of the iron oxide particle 2 and the phospholipid 4 or the amphiphilic polymer 6 is dried and then supplemented with water, and the resulting solution is emulsified to obtain a micelle. In the nanoemulsion method, a mixed solution of the first liquid 10 and the second liquid 11 is emulsified to obtain an oil-in-water (O/W) type emulsion.

The emulsion and the micelle encompass an emulsion and a micelle of any physical property within a range that can achieve the object of the present embodiment. An emulsion and a micelle having one-peak particle size distribution can be used.

Such an emulsion or a micelle can be prepared by a conventional emulsification approach known in the art. The conventional method known in the art is, for example, intermittent shaking, stirring using a mixer such as a propeller-type stirrer or a turbine-type stirrer, a colloid mill method, a homogenizer method, and ultrasonic wave irradiation. These methods may be used alone or in combination of two or more thereof. Moreover, the emulsion and the micelle may be prepared by one-step emulsification or by multi-step emulsification. However, the emulsification approach is not limited to the approach described above within a range that can achieve the object of the present embodiment.

Particularly, the emulsion 12 in the nanoemulsion method is an oil-in-water (O/W) type emulsion prepared from the mixed solution of the first liquid 10 and the second liquid 11. In this context, the mixing of the first liquid 10 and the second liquid 11 means that the first liquid 10 and the second liquid 11 are present in contact with each other without being spatially sequestered from each other. This mixing does not necessarily require being integrated with each other.

The ratio between the first liquid 10 and the second liquid 11 in the mixed solution is not particularly limited as long as the oil-in-water (O/W) type emulsion 12 can be formed. The first liquid and the second liquid can be mixed at a weight ratio ranging from 1:2 to 1:1000.

### (Evaporation)

The evaporation is the procedure of removing the organic solvent contained in the first liquid 10.

The evaporation can be performed by any conventional known method. Examples of the method can include removal by heating and a method using a vacuum apparatus such as an evaporator. In the removal by heating, the heating temperature can range from 0°C to 80°C. However, the evaporation is not limited to the approach described above within a range that can achieve the object of the present embodiment.

### (Ligand molecule)

In the present embodiment, the ligand molecule 8 is a substance arbitrarily selected from chemicals such as biomolecules and pharmaceuticals, for example, a substance specifically binding to a target site such as cancer and a substance specifically binding to a substance present in the neighborhood of the target site. Specific examples thereof include antibodies, antibody fragments, enzymes, bioactive peptides, glycopeptides, sugar chains, lipids, and molecular recognition compounds.

In the present embodiment, the ligand molecule 8 is chemically bound to the contrast agent 1 for photoacoustic imaging having the phospholipid 4 or the amphiphilic polymer 6 to constitute the contrast agent 9 for photoacoustic imaging according to the present embodiment. Use of such a contrast agent 9 for photoacoustic imaging can achieve the specific detection of a target site or the monitoring of pharmakokinetics, localization, drug efficacy, and metabolism of a target substance.

### (Immobilization of ligand molecule)

The ligand molecule 8 can be immobilized onto the contrast agent 1 for photoacoustic imaging having the phospholipid 4 to obtain the contrast agent 9 for photoacoustic imaging of the present embodiment.

In the present embodiment, chemical binding through the reaction between the functional group of the phospholipid 4 or the amphiphilic polymer 6 and the functional group of the ligand molecule 8 can be used as a method for immobilizing the ligand molecule 8 onto the contrast agent 1 for photoacoustic imaging having the phospholipid 4 or the amphiphilic polymer 6.

Specifically, when the functional group of the phospholipid 4 or the amphiphilic polymer 6 is an N-hydroxysuccinimide group, the ligand molecule 8 can be immobilized onto the contrast agent 1 for photoacoustic imaging through the reaction of the N-hydroxysuccinimide group with the amino group of the ligand molecule 8. After the immobilization of the ligand molecule 8, the unreacted N-hydroxysuccinimide group of the phospholipid 4 or the amphiphilic polymer 6 can be inactivated through the reaction of the succinimide group with glycine, ethanolamine, or oligo(ethylene glycol) or polyethylene glycol having a terminal amino group.

Alternatively, when the functional group of the phospholipid 4 or the amphiphilic polymer 6 is a maleimide group, the ligand molecule 8 can be immobilized onto the contrast agent 1 for photoacoustic imaging through the reaction of the maleimide group with the thiol group of the ligand molecule 8. After the immobilization of the ligand molecule 8, the unreacted maleimide group of the phospholipid 4 or the amphiphilic polymer 6 can be inactivated through the reaction of the maleimide group with L-cysteine, mercaptoethanol, or oligoethylene glycol or polyethylene glycol having a terminal thiol group.

Alternatively, when the functional group of the phospholipid 4 or the amphiphilic polymer 6 is an amino group, the ligand molecule 8 can be immobilized onto the contrast agent 1 for photoacoustic imaging through the reaction of the amino group with the amino group of the ligand molecule 8 using glutaraldehyde. After the immobilization of the ligand molecule 8, the activity of the aldehyde group can be blocked through the reaction with ethanolamine or oligo(ethylene glycol) or polyethylene glycol having a terminal amino group.

Alternatively, the amino group may be substituted by an N-hydroxysuccinimide or maleimide group, via which the ligand molecule 8 is immobilized.

### (Imaging using contrast agent for photoacoustic imaging)

The contrast agents 1 and 9 for photoacoustic imaging according to the present embodiment can be used as contrast agents for photoacoustic imaging using near-infrared light.

The contrast agents 1 and 9 for photoacoustic imaging can be dispersed, for use, in a solvent such as saline or injectable distilled water. Moreover, a pharmacologically acceptable additive may be added appropriately thereto, if necessary. These contrast agents for photoacoustic imaging can be introduced into a living body through intravenous or hypodermic injection.

Hereinafter, the present invention will be described more specifically with reference to Examples. However, the present invention is not limited to these Examples. Materials, composition conditions, reaction conditions, and so on can be changed arbitrarily within a range that produces a contrast agent for photoacoustic imaging having the same functions or effect thereas.

### (Photoacoustic imaging method)

A photoacoustic imaging method according to the present embodiment includes the followings:
(i) irradiating, with light in a wavelength region of 600 nm to 1300 nm, a sample that has received the contrast agent for photoacoustic imaging; and
(ii) detecting an acoustic wave generated from the contrast agent present in the sample.
In this context, an apparatus for detecting the acoustic wave is not particularly limited. For example, an ultrasonic probe can be used.

### Examples

In Examples below, specific reagents and reaction conditions used in a contrast agent for photoacoustic imaging of the present invention are listed. However, these reagents or reaction conditions may be changed, and these changes are incorporated in the scope of the present invention. Thus, Examples below are intended to help understand the present invention and do not limit the scope of the present invention by any mans.

### (Preparation of particle containing iron oxide particle)

A large number of particles containing an iron oxide particle are commercially available, and those skilled in the art can easily obtain and use an appropriate one. Also, a large number of documents disclose a method for producing a particle containing an iron oxide particle. Thus, the particle containing an iron oxide particle can be synthesized easily with reference to these documents. For example, an aqueous solution (600 ml) containing FeCl₃·6H₂O (25.5 g) and FeCl₂·4H₂O (10.2 g) mixed and dissolved therein, powdery dextran (molecular weight: 10000 daltons, 360 g), and a 30% NH₄OH solution (30 ml) can be used to prepare a colloidal solution of a dextran particle containing an iron oxide particle according to the method of U.S. Patent No. 5262176. Moreover, various linker molecules can also be bound to the surface of the particle containing an iron oxide particle. For example, a carbodiimide condensing agent 1-ethyl-3-(3-dimethylaminopropyl)carbodiimide hydrochloride (3.6 mg) and 3,6-dioxaoctanedioic acid (3.6 mg) are dissolved in 0.5 M β-morpholinoethanesulfonic acid buffer (1.25 ml, pH=6.3) and incubated at 50°C for 10 minutes. The solution is added to the dextran particle suspension and reacted at room temperature for 2 hours. The particle containing an iron oxide particle can be purified using a magnet to obtain a particle containing an iron oxide particle with oligoethylene oxide as a linker molecule having a terminal carboxyl group.

### (Preparation of single-chain antibody hu4D5-8scFv)

Based on the gene sequence (hu4D5-8) of a HER2-binding IgG variable region, a gene hu4D5-8scFv encoding a single-chain antibody (scFv) was prepared. First, cDNA including the VL and VH genes of hu4D5-8 linked via cDNA encoding a peptide (GGGGS)₃ was prepared. NcoI- and NotI restriction sites were introduced to the 5' and 3' ends thereof, respectively. The nucleotide sequence is shown below.

5'-
CCATGGATATCCAGATGACCCAGTCCCCGAGCTCCCTGTCCGCCTCTGTGGGCGATAGG
GTCACCATCACCTGCCGTGCCAGTCAGGATGTGAATACTGCTGTAGCCTGGTATCAACA

GAAACCAGGAAAAGCTCCGAAACTACTGATTTACTCGGCATCCTTCCTCTACTCTGGAG
TCCCTTCTCGCTTCTCTGGATCCAGATCTGGGACGGATTTCACTCTGACCATCAGCAGT
CTGCAGCCGGAAGACTTCGCAACTTATTACTGTCAGCAACATTATACTACTCCTCCCAC
GTTCGGACAGGGTACCAAGGTGGAGATCAAAGGCGGTGGTGGCAGCGGTGGCGGTGGCA
GCGGCGGTGGCGGTAGCGAGGTTCAGCTGGTGGAGTCTGGCGGTGGCCTGGTGCAGCCA
GGGGGCTCACTCCGTTTGTCCTGTGCAGCTTCTGGCTTCAACATTAAAGACACCTATAT
ACACTGGGTGCGTCAGGCCCCGGGTAAGGGCCTGGAATGGGTTGCAAGGATTTATCCTA
CGAATGGTTATACTAGATATGCCGATAGCGTCAAGGGCCGTTTCACTATAAGCGCAGAC
ACATCCAAAAACACAGCCTACCTGCAGATGAACAGCCTGCGTGCTGAGGACACTGCCGT
CTATTATTGTTCTAGATGGGGAGGGGACGGCTTCTATGCTATGGACTACTGGGGTCAAG
GAACCCTGGTCACCGTCTCCTCGGCGGCCGC-3′ (SEQ ID NO: 1)

(Restriction sites are underlined.)

The gene fragment hu4D5-8scFv was inserted downstream of a T7/lac promoter of a plasmid pET-22b (+) (Novagen, Inc.). Specifically, the cDNA was ligated with pET-22b (+) (Novagen, Inc.) digested with restriction enzymes NcoI- and NotI.

*Escherichia coli* BL21(DE3) was transformed with this expression plasmid to obtain a bacterial strain for expression. The obtained bacterial strain was precultured overnight in 4 ml of LB-Amp medium. Then, the whole amount thereof was added to 250 ml of 2xYT medium and shake-cultured at 120 rpm at 28°C for 8 hours. Then, after addition of IPTG (isopropyl-β-D(-)-thiogalactopyranoside) at a final concentration of 1 mM, the bacterial strain was cultured overnight at 28°C. The cultured *Escherichia coli* was centrifuged at 8000×g at 4°C for 30 minutes, and the supernatant of the culture solution was collected. To the obtained culture solution, 60% by weight of ammonium sulfate was added, and proteins were precipitated by salting-out. The solution subjected to salting-out was left standing overnight at 4°C and then centrifuged at 8000×g at 4°C for 30 minutes to collect precipitates. The obtained precipitates were dissolved in 20 mM Tris·HCl/500 mM NaCl buffer and dialyzed against 1 l of the same buffer. The protein solution thus dialyzed was added to a column charged with His·Bind (registered trademark) Resin (Novagen, Inc.) and purified by metal chelate affinity chromatography via Ni ions. The purified hu4D5-8scFv was confirmed in reducing SDS-PAGE to exhibit a single band and have a molecular weight of approximately 28 kDa. The amino acid sequence of the prepared antibody is shown below. Hereinafter, the hu4D5-8scFv is also abbreviated to scFv.

DIQMTQSPSSLSASVGDRVTITCRASQDVNTAVAWYQQKPGKAPKLLIYSASFLYS
GVPSRFSGSRSGTDFTLTISSLQPEDFATYYCQQHYTTPPTFGQGTKVEIKGGGGS
GGGGSGGGGSEVQLVESGGGLVQPGGSLRLSCAASGFNIKDTYIHWVRQAPGKGLE
WVARIYPTNGYTRYADSVKGRFTISADTSKNTAYLQMNSLRAEDTAVYYCSRWGGD
GFYAMDYWGQGTLVTVSSAAALEHHHHHHGGC
(SEQ ID NO: 2)

### (Binding between single-chain antibody hu4D5-8scFv and iron oxide particle)

The buffer of the scFv thus prepared was replaced by a phosphoric acid buffer containing 5 mM EDTA (2.68 mM KCl/137 mM NaCl/1.47 mM KH₂PO₄/1 mM Na₂HPO₄/5 mM EDTA, pH 7.4). Then, the scFv was reduced at 25°C for approximately 2 hours using a 10-fold molar amount of tris(2-carboxyethyl)phosphine (hereinafter, also abbreviated to TCEP). This scFv thus reduced was reacted at 25°C for approximately 2 hours with nanomag (registered trademark)-D-spio (manufactured by Micromod Partikeltechnologie GmbH; average particle size: 20 nm), a maleimide-surface modified particle containing an iron oxide particle (hereinafter, abbreviated to NP-Maleimide). The reaction was performed at a reaction molar ratio of feed(scFv/particle containing an iron oxide particle) of 1, 4, 10, and 15. In this context, the "feed" mean materials added to the reaction system. The "reaction molar ratio of feed" refers to the ratio by molar concentration between the scFv and the particle containing an iron oxide particle added to the reaction system. After the reaction, scFv unbound to the particle containing an iron oxide particle was removed by ultrafiltration using Amicon Ultra-4 (Nihon Millipore K.K.) having a pore size of 100 kDa to obtain a complex of the scFv and the particle containing an iron oxide particle. After the ultrafiltration, the amount of the scFv immobilized on the particle containing an iron oxide particle was calculated by quantifying the unreacted scFv contained in the filtrate. Moreover, the particle yield was determined from the absorbance of the sample at 490 nm using a standard curve of a solution of a particle containing an iron oxide particle with a known concentration. Hereinafter, the obtained particle is abbreviated to scFv-NP. The average hydrodynamic diameter of the scFv-NP was determined by dynamic light scattering to be 30 to 40 nm (number-average distribution).

### (Binding between antibody and iron oxide particle)

An antibody (also abbreviated to IgG) was bound to a particle containing an iron oxide particle by the carbodiimide method. 20 mg of 1-ethyl-3-(3-dimethylaminopropyl)carbodiimide hydrochloride and 24 mg of N-hydroxysuccinimide were dissolved in 1 ml of 0.5 M β-morpholinoethanesulfonic acid buffer (pH=6.3). Next, the solution was added to 7 ml of a particle suspension containing nanomag (registered trademark)-D-spio COOH (manufactured by Micromod Partikeltechnologie GmbH; average particle size: 20 nm) (hereinafter, abbreviated to NP-COOH) at a particle concentration of 5 mg/ml. This particle suspension was stirred at room temperature for 1 hour. Then, the activated carboxyl group-containing particle containing an iron oxide particle was separated from the low-molecular-weight reagent using a PD-10 desalting column (manufactured by GE Healthcare Biosciences). The developing solvent used was a sodium carbonate buffer (pH 8.0), and buffer exchange was performed simultaneously therewith. Next, to this particle suspension, Herceptin (registered trademark) (manufactured by Chugai Pharmaceutical Co., Ltd.) (trastuzumab) was added as an anti-HER2 antibody. The reaction was performed at a reaction molar ratio of feed (IgG/particle containing an iron oxide particle) of 1, 4, 10, and 20. This particle suspension was stirred at room temperature for 4 hours. Then, after addition of a 1 M aqueous glycine solution at a final glycine concentration of 1 mM, the mixture was stirred at room temperature for 30 minutes to block residual active sites on the surface of the particle containing an iron oxide particle. The obtained Herceptin (registered trademark)-immobilized particle containing an iron oxide particle was purified by gel filtration chromatography (Superdex 200GL10/300 column, manufactured by GE Healthcare Biosciences). The developing solvent used was phosphate-buffered saline (PBS, pH=7.4). The Herceptin (registered trademark)-immobilized particle containing an iron oxide particle was eluted into a void volume fraction, which was then collected. The amount of the IgG immobilized on the particle containing an iron oxide particle was calculated by quantifying the concentration of the unreacted antibody from the peak area derived therefrom. Moreover, the particle yield was determined from the absorbance of the sample at 490 nm using a standard curve of a solution of a particle containing an iron oxide particle with a known concentration. The particle yield (the amount of particles collected relative to the amount of feeded particles) was 20 to 40%. Hereinafter, the Herceptin (registered trademark)-immobilized particle containing an iron oxide particle is referred to as IgG-NP. The average hydrodynamic diameter of the IgG-NP was determined by dynamic light scattering to be 50 to 60 nm (number-average distribution).

### (Binding between antibody and iron oxide particle having ethylene oxide linker)

An antibody was bound to a particle containing an iron oxide particle with an ethylene oxide linker according to the paragraph "Binding between antibody and iron oxide particle". The same procedures as in the paragraph "Binding between antibody and iron oxide particle" were performed except that the particle containing an iron oxide particle used was nanomag (registered trademark)-D-spio COOH-PEG (manufactured by Micromod Partikeltechnologie GmbH; average particle size: 20 nm, PEG linker molecular weight: 300) (hereinafter, abbreviated to NP-EO-COOH). The particle yield (the amount of particles collected relative to the amount of feed particles) was 70 to 80%. Hereinafter, the Herceptin (registered trademark)-immobilized particle containing an iron oxide particle is referred to as IgG-EO-NP. The average hydrodynamic diameter of the IgG-EO-NP was determined by dynamic light scattering to be 30 to 60 nm (number-average distribution). Moreover, the particle had a zeta potential of -4.5 mV in PBS.

### (Binding between control antibody and iron oxide particle)

A control antibody was bound to a particle containing an iron oxide particle according to the paragraph "Binding between antibody and iron oxide particle having ethylene oxide linker". In this context, the control antibody used was purified human IgG (polyclonal, manufactured by Dainippon Sumitomo Pharma Co., Ltd.). The reaction was performed at a reaction molar ratio of feed (IgG/particle containing an iron oxide particle) of 10. The amount of the IgG immobilized on the particle containing an iron oxide particle was 4 (IgG/particle containing an iron oxide particle). Hereinafter, the control antibody-immobilized particle containing an iron oxide particle is referred to as cIgG-NP.

### Example 1

### (Characteristic evaluation of antibody-immobilized iron oxide particle)

The amounts of the antibody immobilized, binding efficiencies, antibody immobilization densities, and particle yields of the antibody-immobilized iron oxide particles thus prepared are summarized in Table 1.

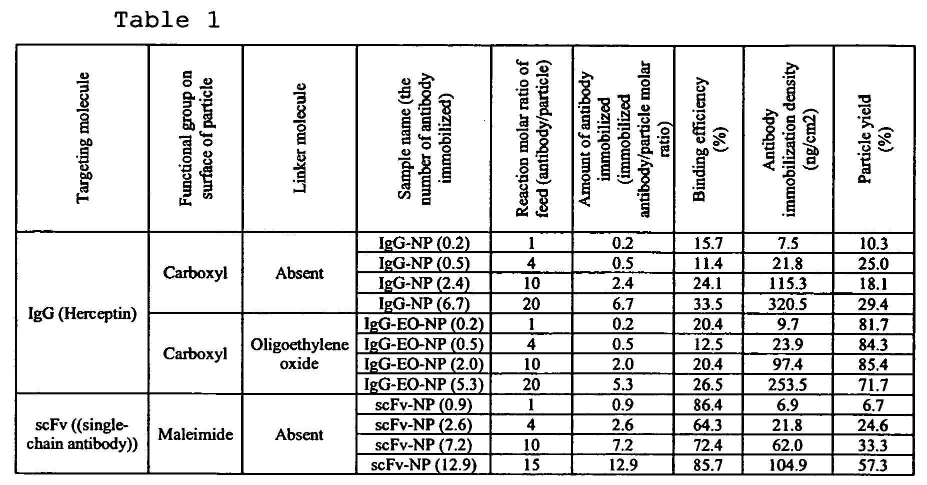

The amount of the antibody immobilized could be increased by increasing the feed ratio of the antibody to the iron oxide particle. In the IgG-NP and the IgG-EO-NP having IgG as a ligand molecule, the amount of the antibody immobilized was very low relative to the feed ratio. Specifically, their binding efficiencies were as low as 11 to 33%. On the other hand, when the ligand molecule was scFv, the binding efficiency was very favorable (64 to 85%). In this context, the iron oxide particle used had an average particle size of 20 nm, and the maximum amount of IgG immobilized is estimated from the surface area to be approximately 8 to 10 molecules (IgG is hypothesized as a spherical molecule of 10 nm in diameter). Thus, binding efficiency of 50 to 70% may be correct for the IgG systems having the reaction molar ratio of feed of 20.

Moreover, referring to the particle yield (the amount of particles collected relative to the amount of feeded particles), the antibody-immobilized iron oxide particles having oligoethylene oxide (molecular weight: 300) as a linker molecule exhibited a yield as high as 71 to 85%. On the other hand, the IgG-NP free from the linker molecule had a yield reduced to 10 to 30%. This may be because: the immobilization reaction of IgG to particles causes aggregation among the particles due to the antibody molecule serving as a binder; or the denaturation of the immobilized IgG on the particle surface triggers aggregation among the particles. Similar phenomena seem to occur in the scFv-NP. In actuality, the IgG-NP and scFv-NP systems were confirmed to produce aggregated particle precipitates after antibody immobilization reaction. On the other hand, the IgG-EO-NP having an oligoethylene oxide linker probably has favorable dispersibility among the particles, leading to suppressed aggregation among the particles. Interestingly, in the scFv-NP systems, the particle yield was increased with increases in the feed ratio between the antibody and the particle. This might be because the maleimide group-containing particle containing an iron oxide particle was unstable, and the immobilization of large number of scFv fragments improved the dispersibility of the particle.

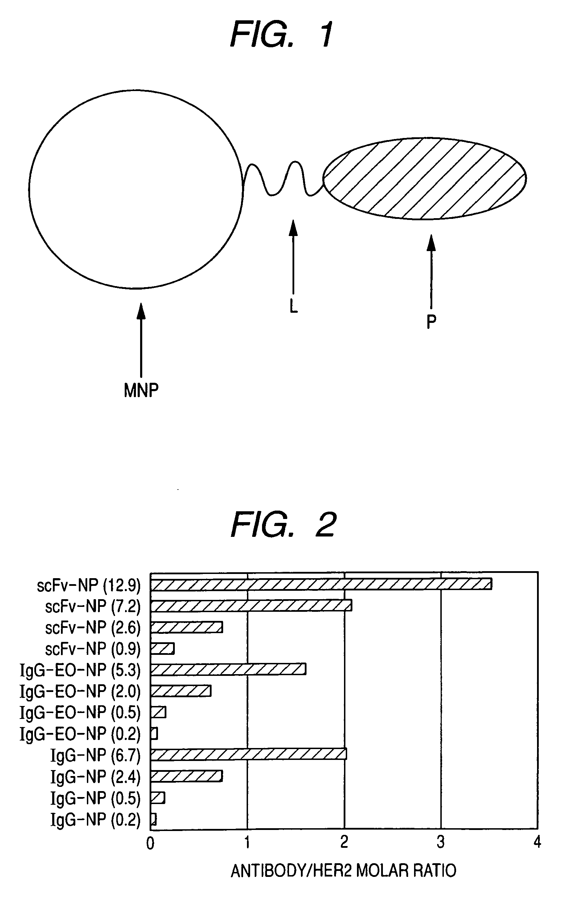

The antibody immobilization density was 7.5 to 320.5 ng/cm² for the IgG systems and 6.9 to 104.9 ng/cm² for the scFv systems. In this context, the cell surface density of HER2 is calculated. First, when the number of HER2 in HER2-positive cells (strongly expressing strain) is defined as 10⁶ molecules/cell and the cell diameter is defined as 10 µm, the cell surface density of HER2 is calculated to be 1 pmol/cm². The antibody immobilization densities are also separately converted to a molar density, which is then divided by the cell surface density of HER2 (1 pmol/cm²) to calculate the number of the antibody per HER2 molecule (antibody/HER2 molar ratio). This result is illustrated in FIG. 2. The (antibody/HER2 molar ratio) needs to be at least 1 or larger for achieving sufficient multivalent binding to HER2. From the results of FIG. 2, multivalent interaction with HER2 can be expected sufficiently in the scFv-NP (12.9), suggesting that strong binding capability can be achieved.

### Example 2

### (Binding specificity evaluation of antibody-immobilized iron oxide particle)

The antibody-immobilized iron oxide particles prepared were evaluated for their binding activities and binding specificities for HER2 by fluorescent immunostaining. The cells used were N87 as HER2-positive cells (HER2+). The HER2-negative cells (HER2-) used were MDA-MB-231. The test particles used were IgG-NP (6.7), IgG-EO-NP (5.3), scFv-NP (12.9), and cIgG-NP. Herceptin (registered trademark) was used as a positive control. The detection antibodies (fluorescently labeled antibodies) used were an Alexa Fluor (registered trademark) 488-labeled anti-human IgG antibody (for Herceptin (registered trademark), IgG-NP, IgG-EO-NP, or cIgG-NP detection), an anti-His tag antibody (mouse), and an Alexa Fluor (registered trademark) 488-labeled antimouse IgG antibody (for scFv-NP detection). Each cell was cultured overnight in a 24-well plate and then fixed in 5% paraformaldehyde (PFA). The cells were washed with PBS. Then, after addition of Herceptin (registered trademark) or each antibody-immobilized iron oxide particle, the mixture was incubated at 37°C for 1 hour in a growth medium. Then, the cells were washed with a growth medium and then incubated with the detection antibody (1 hr, 37°C). After washing, the cells were observed under a fluorescence microscope.

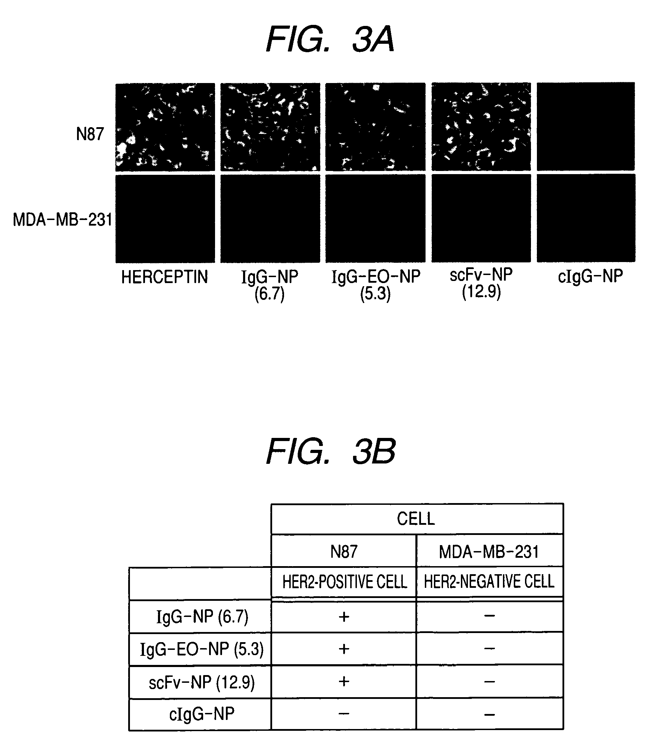

The results of the fluorescent immunostaining are illustrated in FIGS. 3A and 3B. FIG. 3A illustrates the fluorescent images of the HER2-positive cells (N87, upper boxes) and the HER2-negative cells (MDA-MB-231, lower boxes) obtained using the antibody-immobilized iron oxide particles of the present invention. In the diagram, the fluorescence indirectly represents the presence of the antibody as a ligand molecule, i.e., the presence of the antibody-immobilized iron oxide particle. As is evident from the staining results of Herceptin (registered trademark) as a positive control, it was confirmed that HER2 was expressed in N87, whereas HER2 was expressed in MDA-MB-231, albeit at a sufficiently low level that could not be detected by this experimental system. FIG. 3B is a table of the staining results of FIG. 3A. In the table, + represents stainable, and - represents unstainable. The antibody-immobilized iron oxide particles prepared were confirmed to bind to the HER2-positive cell N87. Moreover, the cIgG-NP did not bind to N87, demonstrating that the binding of the particles was not nonspecific. On the other hand, the HER2-negative cell MDA-MB-231 was not stained with any of the particles, demonstrating that the antibody-immobilized iron oxide particle of the present invention has binding specificity for HER2.

### Example 3

### (Photoacoustic signal measurement of antibody-immobilized iron oxide particle)

The antibody-immobilized iron oxide particles prepared were evaluated for their photoacoustic signal transmission abilities. The test particles used were IgG-NP (6.7), IgG-EO-NP (5.3), scFv-NP (12.9), and cIgG-NP. Moreover, the photoacoustic signal transmission abilities of antibody-non-immobilized iron oxide particles (NP-Maleimide, NP-COOH, and NP-EO-COOH) were also determined. Each particle was subjected to photoacoustic signal measurement at at least 3 concentrations. PBS was used as a solvent. The absorbance at 710 nm was measured before and after the photoacoustic signal measurement. As a result, the difference therebetween was within 5%. The magnetite content and the particle size of the iron oxide particle (i.e., the particle size of the core particle) were the same among all the particles.

In photoacoustic measurement, the sample dispersed in PBS was irradiated with pulse laser light. Photoacoustic signals from the sample were detected using a piezoelectric element, amplified using a high-speed preamplifier, and then obtained using a digital oscilloscope. Specific conditions are as follows: the optical source used was a titanium-sapphire laser (LT-2211-PC, manufactured by LOTIS LTD.). The conditions involved a wavelength of 710 nm, an energy density of approximately 10 to 20 mJ/cm2, a pulse width of approximately 20 nanoseconds, and a pulse repetition frequency of 10 Hz. The piezoelectric element for detecting photoacoustic signals was a non-convergence-type ultrasonic transducer (V303, manufactured by Panametrics-NDT) having an element diameter of 1.27 cm and a central frequency of 1 MHz. The measurement vessel was a poly(styrene) cuvette having an optical path length of 0.1 cm and a sample volume of approximately 200 µl.

The measurement vessel and the piezoelectric element were dipped at a distance of 2.5 cm therebetween in a glass container filled with water. The high-speed preamplifier used for amplifying photoacoustic signals was an ultrasonic preamplifier (Model 5682, manufactured by OLYMPUS CORP.) having an amplification degree of +30 dB. The amplified signals were input to a digital oscilloscope (DPO4104, manufactured by Tektronix). The poly(styrene) cuvette was irradiated with pulse laser light from outside of the glass container. A portion of light scattered therefrom was detected using a photodiode and input as a trigger signal to the digital oscilloscope. The digital oscilloscope was set to a 32 run-averaging display mode to obtain an average photoacoustic signal of 32 laser pulse irradiations.

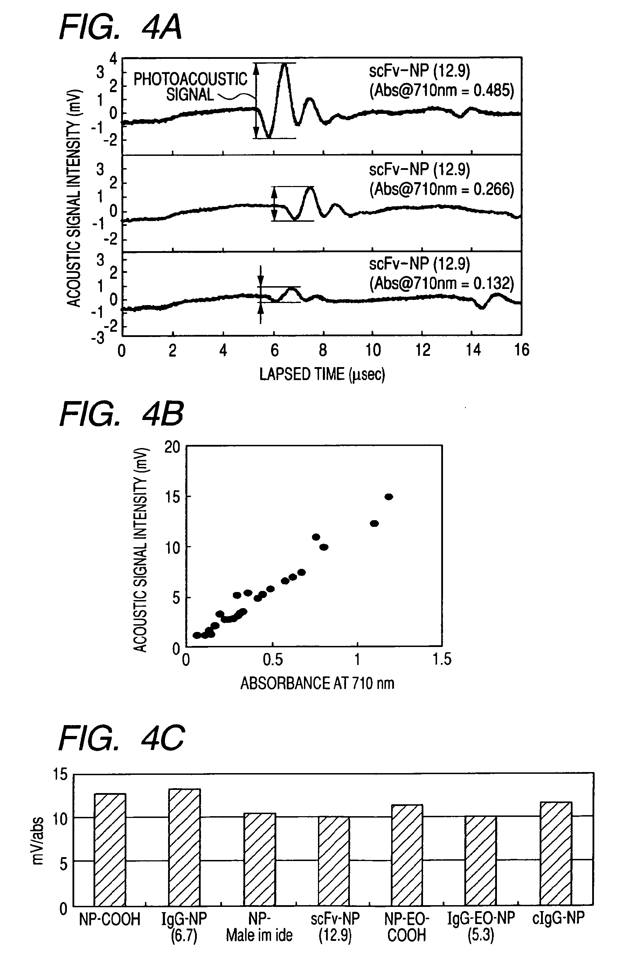

The results of measuring photoacoustic signals are illustrated in FIGS. 4A to 4C. FIG. 4A illustrates, as a typical example, the photoacoustic signal waveform of the scFv-NP (12.9) obtained in the oscilloscope. The graph represents a list of the results of the scFv-NP (12.9) solution at varying absorbances (i.e., varying scFv-NP concentrations). The peaks detected later may be influenced by reflections in the cell. Therefore, only the first peak is effective as a signal. As is evident from FIG. 4A, a photoacoustic signal was confirmed from the test particle. Moreover, the absorbance dependence of the scFv-NP (12.9) solution in photoacoustic signal intensity was confirmed. Likewise, photoacoustic signal transmission and absorbance dependence of signal intensity could also be confirmed in all of the other test particles. FIG. 4B illustrates a plot of the photoacoustic signal intensities of all the test particles vs. absorbance at 710 nm.

From FIG. 4B, it was confirmed that the photoacoustic signal has a linear relationship with the absorbance of the test particle solution. Moreover, photoacoustic signal intensity per unit absorbance (mV/abs) was determined from an approximate line of data on the signal intensity of each test particle and absorbance at 710 nm and graphed in FIG. 4C. The value of mV/abs represents the photoacoustic signal transmission ability of each particle. This means that a particle having a higher value of mV/abs is more suitable as a contrast agent for photoacoustic imaging. From FIG. 4C, it was confirmed that: the photoacoustic signal transmission ability does not change between before and after antibody immobilization (in the diagram, compare NP-COOH with IgG-NP, NP-Maleimide with scFv-NP, and NP-EO-COOH with IgG-EO-NP or cIgG-NP); and the photoacoustic signal transmission ability does not differ between IgG and scFv (compare IgG-NP with scFv-NP). These results demonstrated that the antibody-immobilized iron oxide particle of the present invention can immobilize thereon the ligand molecule without reducing the photoacoustic signal transmission ability of the iron oxide particle as a signal-transmitting part and functions as a contrast agent for photoacoustic imaging having high sensitivity and binding capability.

### Example 4

### (Binding between antibody and 20-nm iron oxide particle having ethylene oxide linker)

An antibody (Herceptin (registered trademark)) was bound to a 20-nm iron oxide particle according to the paragraph "Binding between antibody and iron oxide particle having ethylene oxide linker". The reaction was performed at a reaction molar ratio of feed (antibody/particle containing an iron oxide particle) of 20. The amount of the antibody immobilized on the particle containing an iron oxide particle was 5.4 (antibody/particle containing an iron oxide particle). Hereinafter, the Herceptin (registered trademark)-immobilized particle containing an iron oxide particle is referred to as IgG-EO-NP (5.4). The average hydrodynamic diameter of the IgG-EO-NP (5.4) was determined by dynamic light scattering to be 39.4 nm (number-average distribution). Moreover, the particle had a zeta potential of -4.5 mV in PBS.

### Example 5

### (Binding between artificial antibody and 20-nm iron oxide particle having ethylene oxide linker)

An artificial antibody scFv was bound to a particle containing an iron oxide particle with an ethylene oxide linker according to the paragraph "Binding between single-chain antibody hu4D5-8scFv and iron oxide particle". The particle containing an iron oxide particle used was maleimide-surface modified nanomag (registered trademark)-D-spio (manufactured by Micromod Partikeltechnologie GmbH; average particle size: 20 nm, PEG linker molecular weight: 300) (hereinafter, abbreviated to NP-EO-Maleimide-20). The same procedures as in the paragraph "Binding between single-chain antibody hu4D5-8scFv and iron oxide particle" were performed except that the NP-EO-Maleimide-20 was used. The reaction was performed at a reaction molar ratio of feed (scFv/particle containing an iron oxide particle) of 20. The particle yield was 74%, which was higher than the particle yield (57% at maximum) obtained using NP-Maleimide free from the ethylene oxide linker. This indicates that use of the ethylene oxide linker improved the dispersion stability of the particle, as with the results of IgG-EO-NP. Hereinafter, the scFv-immobilized particle containing an iron oxide particle thus obtained is referred to as scFv-EO-NP-20. The average hydrodynamic diameter of the scFv-EO-NP-20 was determined by dynamic light scattering to be 22.3 nm (number-average distribution). The amount of the scFv immobilized on the particle containing an iron oxide particle was 12.2 (scFv/particle). Moreover, the particle had a zeta potential of -4.6 mV in PBS.

### Example 6

### (Preparation of peptide-immobilized iron oxide particle)

### (Binding between HER2-binding peptide and iron oxide particle)

A HER2-binding peptide (amino acid sequence: MARSGLGGKGSC; hereinafter, abbreviated to HBP) was dissolved in a phosphoric acid buffer (2.68 mM KCl/137 mM NaCl/1.47 mM KH₂PO₄/1 mM Na₂HPO₄/5 mM EDTA, pH 7.4) and reacted with the NP-EO-Maleimide-20 at 4°C for approximately 16 hours. The reaction was performed at a reaction molar ratio of feed (HBP/particle containing an iron oxide particle) of 71. After the reaction, HBP unbound to the particle containing an iron oxide particle was removed by ultrafiltration using Amicon Ultra-4 (Nihon Millipore K.K.) having a pore size of 10 kDa to obtain a complex of the HBP and the particle containing an iron oxide particle. After the ultrafiltration, the amount of the HBP immobilized on the particle containing an iron oxide particle was calculated by quantifying the unreacted HBP contained in the filtrate. The amount of the HBP immobilized on the particle containing an iron oxide particle was 47 (HBP/particle containing an iron oxide particle). Hereinafter, the HBP-immobilized particle containing an iron oxide particle thus obtained is referred to as HBP-EO-NP-20. The average hydrodynamic diameter of the HBP-EO-NP-20 was determined by dynamic light scattering to be 28.1 nm (number-average distribution). Moreover, the particle had a zeta potential of -0.6 mV in PBS. In this context, the HER2-binding peptide used was designed based on a HER2-binding peptide MARSGL reported in Int. J. Cancer, 92, 748-755 (2001). This peptide was linked to a linker sequence GGKGSC and bound to the particle using the side chain thiol of the C-terminal cysteine.

### Example 7

### (Evaluation of HER2 binding capability of ligand molecule-immobilized iron oxide particle)

The interaction between each particle and HER2 was analyzed using Biacore X system (GE Healthcare Japan Corp.). The antigen used was Recombinant Human ErbB2/Fc Chimera (R&D Systems, Inc.). The antigen was immobilized by amine coupling to a carboxymethyldextran chain on the surface of a CM-5 chip according to the manufacturer's recommended protocol. The amount of the antigen immobilized was approximately 1000 to 2000 RU. PBS-T (2.68 mM KCl/137 mM NaCl/1.47 mM KH2PO4/1 mM Na2HP04/0.005% Tween 20, pH 7.4) was used as a running buffer. The particle concentration was set to 0.1 to 100 nM in terms of the scFv concentration. The flow rate was set to 2 to 4 µl/min, and the binding time was set to 10 to 20 min. The binding amount of the particle applied at each concentration, i.e., response Req, was calculated. A Scatchard plot was prepared, and an apparent equilibrium dissociation constant (Kd) was determined from the slope. As a result, the equilibrium dissociation constants (Kd) of the IgG-EO-NP (5.4), the scFv-EO-NP-20, and the HBP-EO-NP-20 for HER2 were 0.6 nM, 0.01 nM, and 36.7 nM, respectively. The strongest binding capability was obtained in the scFv-immobilized particle.

### Example 8

### (Evaluation of binding capability of ligand molecule-immobilized particle containing iron oxide particle to HER2-expressing cells)

Each particle was evaluated for its binding capability to HER2-expressing cells. On the day before evaluation, HER2-positive cells (N87 cells) were seeded over a 24-well plate (5×10⁵ cells/well). Next day, after medium removal, 200 µL of a growth medium was placed therein, and the particle sample was then added to each well. The plate was left standing in a CO₂ incubator at 37°C for 4 hours. Then, the medium containing the particle was removed. The wells were fully washed twice with 1 mL of PBS. After removal of PBS, 1% Triton-X100/PBS solution was added at a concentration of 150 µL/well for cell lysis. The cells were incubated at room temperature for 2 hours or longer. Subsequently, concentrated hydrochloric acid (12 N) was added at a concentration of 150 µL/well, and the cells were incubated at room temperature for 2 hours or longer. The amount of iron in this acid lysate of the sample (6 N HCl solution, 300 µL) was measured to determine the concentration of the particle bound to the cell. A Scatchard plot was prepared from the saturation binding curve of the particle for N87, and an apparent equilibrium dissociation constant (Kd) of the particle for the cell was determined. As a result, the equilibrium dissociation constants (Kd) of the IgG-EO-NP (5.4), the scFv-EO-NP-20, and the HBP-EO-NP-20 for N87 cells were 10 nM, 5.8 nM, and 78 nM, respectively. The strongest binding capability was obtained in the scFv-immobilized particle.

### Example 9

### (Evaluation of mouse pharmacokinetics of ligand molecule-immobilized particle containing iron oxide particle)

Each ligand molecule-immobilized iron oxide particle prepared as described above was evaluated for its pharmacokinetics in cancer-bearing mice. The test particles used were IgG-EO-NP (5.4), cIgG-NP, scFv-EO-NP-20, and HBP-EO-NP-20. First, each particle was labeled with a radioisotope (hereinafter, abbreviated to RI) according to a method known in the art. The RI nuclide used was 111-indium (also abbreviated to ¹¹¹In) that is most suitable for pharmacokinetics study from the viewpoint of energy, half-life, and easy radiolabeling. Bifunctional chelating diethylene-triamine-pentaacetic acid (DTPA) anhydride (DTPA anhydride) was used for labeling the particle with ¹¹¹In. First, the DTPA anhydride was reacted with the lysine residue of the antibody or the amino group of the particle, and the reaction product was purified using Sephadex G-50 (manufactured by GE Healthcare Japan Corp.). Then, after labeling with 111-indium, the labeled particle was purified using PD-10 (manufactured by GE Healthcare Japan Corp.) to obtain an RI-labeled particle.

The RI-labeled particle (470 µg/100 µL PBS, radiochemical purity: >90%) was intravenously administered to cancer-bearing mouse models and examined for its pharmacokinetics for 72 hours after administration by evisceration. The time points of evaluation were set to 1, 3, 6, 24, 48, and 72 hours after administration, and 3 or more mice were used at each time point. The cancer-bearing mouse models used were BALB/c nude mice that had received a hypodermic graft of N87 cells highly expressing HER2. More specifically, 5×10⁶ N87 cells were hypodermically injected to the shoulder of each BALB/c Slc-nu/nu mouse (Japan SLC, Inc.). 14 days later, cancer-bearing mouse models having N87 tumor of approximately 4 to 6 mm formed therein were prepared. At this point in time, the particle was administered to the mice. The dose of the particle to the mice was 7.4×10¹³ particles/mouse or 0.22 mg iron/mouse. The radiation dose was 370 kBq/mouse.

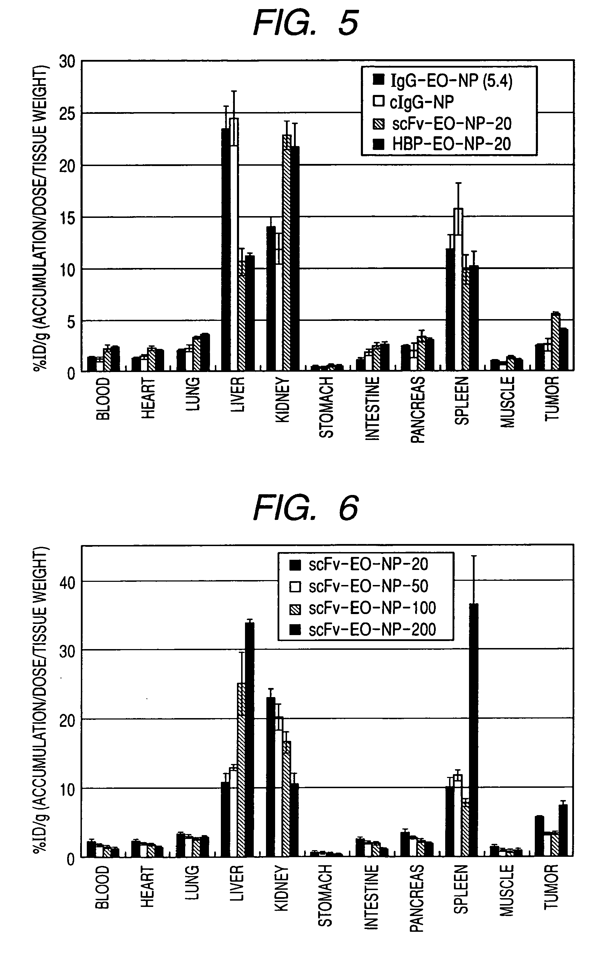

The results of pharmacokinetics at 24 hours after administration are illustrated in FIG. 5. FIG. 5 is a graph illustrating accumulation (value normalized against dose and tissue weight) in each tissue. Tumor (N87 cell) accumulation was highest in the scFv-EO-NP-20 (5.6%ID/g), followed by the HBP-EO-NP-20 (4.0%ID/g), the IgG-EO-NP (5.4) (2.5%ID/g), and the cIgG-NP (2.5%ID/g). All the antibody-immobilized particles (IgG-EO-NP (5.4) and cIgG-NP) exhibited remarkable accumulation to the liver, whereas the artificial antibody- or peptide-immobilized particles (scFv-EO-NP-20 and HBP-EO-NP-20) exhibited remarkable accumulation to the kidney.

The antibody-immobilized particles were susceptible to opsonization due to antibody denaturation resulting from the antibody binding to the particle and the presence of the Fc domain of the antibody. Therefore, most of these particles seemed to migrate to the liver and the spleen. No difference of tumor accumulation between the antibody and the control antibody may indicate that the accumulation of the antibody-immobilized particles is passive accumulation (tumor accumulation caused by EPR effect).

On the other hand, the artificial antibody was immobilized in a site-directed manner on the particle and is less denatured with high binding capability. Use of the artificial antibody also having a small size could suppress migration to the liver and the spleen and improved a serum concentration, compared to the antibody-immobilized particle. This probably led to the improved tumor accumulation of the artificial antibody-immobilized particle. The remarkable migration to the kidney suggests that the artificial antibody released from the particle surface was likely to accumulate thereto. The peptide has a small size and no Fc domain, albeit with low binding capability. Therefore, use of the peptide reduced accumulation to the liver. This probably led to the tumor accumulation improved compared with the antibody-immobilized particle. These results demonstrated the superiority of the artificial antibody as a ligand molecule in the particle containing an iron oxide particle according to the present invention.

### Example 10

### (Binding between artificial antibody and 50-nm iron oxide particle having ethylene oxide linker)

An artificial antibody scFv was bound to a particle containing a 50-nm iron oxide particle with an ethylene oxide linker according to Example 5. The particle containing an iron oxide particle used was nanomag (registered trademark)-D-spio (manufactured by Micromod Partikeltechnologie GmbH; average particle size: 50 nm, PEG linker molecular weight: 300), a maleimide-surface modified particle containing a 50-nm iron oxide particle (hereinafter, abbreviated to NP-EO-Maleimide-50). The same procedures as in Example 4 were performed except that reaction was performed at a reaction molar ratio of feed (scFv/particle containing an iron oxide particle) of 125 using the NP-EO-Maleimide-50. The particle yield was 76%. Hereinafter, the scFv-immobilized particle containing an iron oxide particle thus obtained is referred to as scFv-EO-NP-50. The average hydrodynamic diameter of the scFv-EO-NP-50 was determined by dynamic light scattering to be 54.5 nm (number-average distribution). The amount of the scFv immobilized on the particle containing an iron oxide particle was 91 (scFv/particle). Moreover, the particle had a zeta potential of -0.5 mV in PBS.

### Example 11

### (Binding between artificial antibody and 100-nm iron oxide particle having ethylene oxide linker)

An artificial antibody scFv was bound to a particle containing a 100-nm iron oxide particle with an ethylene oxide linker according to Example 5. The particle containing an iron oxide particle used was nanomag (registered trademark)-D-spio (manufactured by Micromod Partikeltechnologie GmbH; average particle size: 100 nm, PEG linker molecular weight: 300), a maleimide-surface modified particle containing a 100-nm iron oxide particle (hereinafter, abbreviated to NP-EO-Maleimide-100). The same procedures as in Example 4 were performed except that reaction was performed at a reaction molar ratio of feed (scFv/particle containing an iron oxide particle) of 500 using the NP-EO-Maleimide-100. The particle yield was 62%. Hereinafter, the scFv-immobilized particle containing an iron oxide particle thus obtained is referred to as scFv-EO-NP-100. The average hydrodynamic diameter of the scFv-EO-NP-100 was determined by dynamic light scattering to be 114.2 nm (number-average distribution). The amount of the scFv immobilized on the particle containing an iron oxide particle was 375 (scFv/particle). Moreover, the particle had a zeta potential of -3.1 mV in PBS.

### Example 12

### (Binding between artificial antibody and 200-nm iron oxide particle having ethylene oxide linker)

An artificial antibody scFv was bound to a particle containing a 200-nm iron oxide particle with an ethylene oxide linker according to Example 5. The particle containing an iron oxide particle used was nanomag (registered trademark)-D (manufactured by Micromod Partikeltechnologie GmbH; average particle size: 200 nm, PEG linker molecular weight: 300), a maleimide-surface modified particle containing a 200-nm iron oxide particle (hereinafter, abbreviated to NP-EO-Maleimide-200). The same procedures as in Example 4 were performed except that reaction was performed at a reaction molar ratio of feed (scFv/particle containing an iron oxide particle) of 2000 using the NP-EO-Maleimide-200. The particle yield was 80%. Hereinafter, the scFv-immobilized particle containing an iron oxide particle thus obtained is referred to as scFv-EO-NP-200. The average hydrodynamic diameter of the scFv-EO-NP-200 was determined by dynamic light scattering to be 171.7 nm (number-average distribution). The amount of the scFv immobilized on the particle containing an iron oxide particle was 1460 (scFv/particle). Moreover, the particle had a zeta potential of -1.8 mV in PBS.

### Example 13

### (Evaluation of binding capabilities of scFv-NP with varying particle sizes).

The scFv-NP with varying particle sizes was evaluated for its HER2 binding capability according to the method described in Example 7. As a result, the equilibrium dissociation constants (Kd) of the scFv-EO-NP-20, the scFv-EO-NP-50, the scFv-EO-NP-100, and the scFv-EO-NP-200 for HER2 were 0.01 nM, 0.69 nM, 0.36 nM, and 0.05 nM, respectively. Moreover, the scFv-NP with varying particle sizes was evaluated for its binding capability to HER2-expressing cells according to the method described in Example 8. As a result, the equilibrium dissociation constants (Kd) of the scFv-EO-NP-20, the scFv-EO-NP-50, the scFv-EO-NP-100, and the scFv-EO-NP-200 for HER2-expressing cells were 5.8 nM, 7.87 nM, 1.63 nM, and 0.09 nM, respectively. As seen from these results, the strongest binding capability was obtained in the scFv-EO-NP-200.

### Example 14

### (Evaluation of mouse pharmacokinetics of scFv-NP with varying particle sizes)

The scFv-NP with varying particle sizes prepared as described above was evaluated for its pharmacokinetics in cancer-bearing mice. The test particles used were scFv-EO-NP-20, scFv-EO-NP-50, scFv-EO-NP-100, and scFv-EO-NP-200. According to Example 9, each RI-labeled particle was prepared, intravenously administered to cancer-bearing mouse models, and examined for its pharmacokinetics by evisceration. The dose of the particle to the mice was 0.22 mg iron/mouse. The number of the particle administered differs depending on the particle size and was as follows: 7.4×10¹³ particles/mouse, 5.1×10¹² particles/mouse, 7.0×10¹¹ particles/mouse, and 3.2×10¹⁰ particles/mouse for scFv-EO-NP-20, scFv-EO-NP-50, scFv-EO-NP-100, and scFv-EO-NP-200, respectively.

The results of pharmacokinetics at 24 hours after administration are illustrated in FIG. 6. FIG. 6 is a graph illustrating accumulation (value normalized against dose and tissue weight) in each tissue. Tumor (N87 cell) accumulation was highest in the scFv-EO-NP-200 (7.4%ID/g), followed by the scFv-EO-NP-20 (5.6%ID/g), both the scFv-EO-NP-50 and the scFv-EO-NP-100 (3.3%ID/g). The high tumor accumulation of the scFv-EO-NP-200 is probably a result reflecting the high binding capability of the particle to N87 cells. With increases in particle size, accumulation to the liver or the spleen was increased, while accumulation to the kidney was decreased. The serum concentration of the particle was observed to have a decreasing tendency with increases in particle size. The ratio (contrast) between tumor and blood was highest in the scFv-EO-NP-200 (6.3), followed by the scFv-EO-NP-20 (2.5), the scFv-EO-NP-100 (2.3), and the scFv-EO-NP-50 (2.0). The scFv-EO-NP-200 had high tumor accumulation and a low serum concentration and could therefore achieve a high contrast. From the viewpoint of both tumor accumulation and contrasts, it was shown that the scFv-EO-NP-200 according to the present invention is highly practicable as a contrast agent for photoacoustic imaging.

### Example 15

### (Molar absorption coefficient evaluation and photoacoustic signal evaluation of various ligand molecule-immobilized particles containing an iron oxide particle and NP with varying particle sizes)

Each ligand molecule-immobilized particle containing an iron oxide particle prepared was evaluated for its molar absorption coefficient (ε) and photoacoustic signal (also abbreviated to a PA signal). In the molar absorption coefficient measurement, the particle was dispersed in PBS, and the absorbance of the solution at a wavelength of 710 nm was measured for determination. Moreover, the PA signal evaluation was performed according to the method of Example 3. The PA signal was indicated in a value (V/J/nM) of the obtained signal intensity value (voltage: V) normalized against incident laser intensity (J) and particle concentration (nM). The results are summarized in Tables 2 and 3 below. These results, taken together with the results illustrated in FIG. 4C, demonstrated that the molar absorption coefficient and PA signal of the particle depend on the particle size (Table 3) and are independent of the ligand molecule immobilized (Table 2). The molar absorption coefficient and PA signal of the particle were increased with increases in particle size. This indicates that PA signals and molar absorption coefficients are in a proportional relationship.

**Table 2**

| Particle name | Molar absorption coefficient of particles (M⁻¹·cm⁻¹) | PA signal (710nm) |
|---|---|---|
| IgG-EO-NP (5.4) | 1.68×10⁶ | 0.1 |
| scFv-EO-NP-20 | 1.45×10⁶ | 0.1 |
| HBP-EO-NP-20 | 1.44×10⁶ | 0.1 |

**Table 3**

| Particle name | Molar absorption coefficient of particles (M⁻¹·cm⁻¹) | PA signal (710nm) |
|---|---|---|
| scFv-EO-NP-20 | 1.5×10⁶ | 0.1 |
| scFv-EO-NP-50 | 1.5×10⁷ | 1.0 |
| scFv-EO-NP-100 | 1.9×10⁸ | 8.7 |
| scFv-EO-NP-200 | 1.4×10¹⁰ | 254.6 |

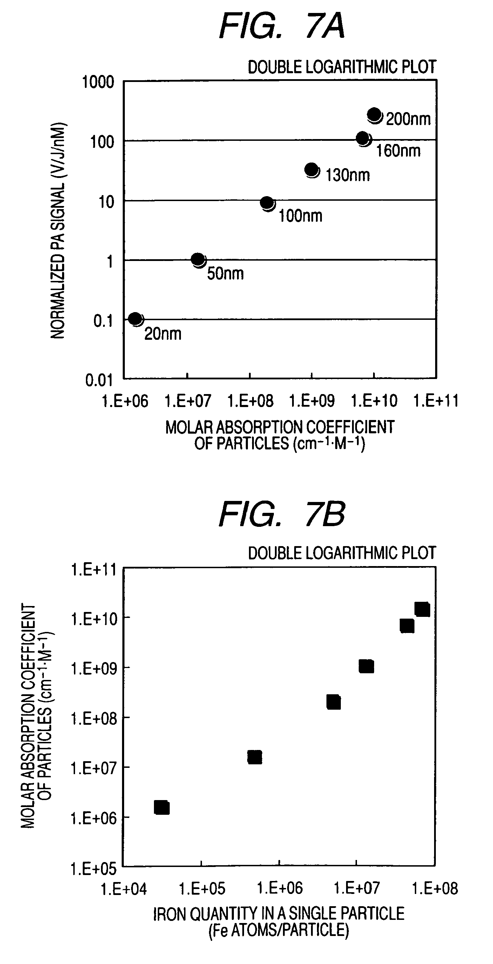

FIG. 7A illustrates the relationship between photoacoustic signals and molar absorption coefficients of particles (double logarithmic plot). In the graph, the numeric values represent particle sizes. In this Example, in addition to the particles prepared above, data on 130-nm and 160-nm iron oxide particles (nanomag (registered trademark)-D, manufactured by Micromod Partikeltechnologie GmbH; average particle size: 130 nm or 160 nm, PEG linker molecular weight: 300) was illustrated. On the other hand, FIG. 7B illustrates the relationship between molar absorption coefficients of particles and iron contents in the particle (the amount of iron per particle) (double logarithmic plot). These graphs demonstrated that PA signal intensity is increased in proportion to the absorption coefficient of the particle, and this absorption coefficient of the particle depends on the amount of iron in the particle. Specifically, for enhancing photoacoustic signals, it is required to increase an iron content in the particle by increasing the particle size and to enhance an absorption coefficient per unit iron oxide (to increase a magnetite content relative to maghemite). The ligand molecule-immobilized particle containing an iron oxide particle according to this Example was rationally designed to solve these problems. A high absorption coefficient is essential for efficiently obtaining the photoacoustic signal of the contrast agent for photoacoustic imaging. For example, the contrast agent having a molar absorption coefficient of particles of 10⁹ (M⁻¹·cm⁻¹) or higher would easily achieve high-contrast imaging. This is supported by a gold nanorod (having a molar absorption coefficient of approximately 10⁹ (M⁻¹·cm⁻¹) in a near-infrared wavelength region) described as a contrast agent for photoacoustic imaging in a large number of previous reports. The ligand molecule-immobilized particle containing an iron oxide particle according to this Example may have an iron content of 10⁷ or more iron atoms for achieving the molar absorption coefficient of 10⁹ (M⁻¹·cm⁻¹) or higher. None of previously reported iron oxide-containing contrast agents for photoacoustic imaging or ligand molecule-immobilized particles containing an iron oxide particle have such a high iron atom content. However, the contrast agent may not require a molar absorption coefficient of particles as high as 10⁹ (M⁻¹·cm⁻¹), depending on its usage or accumulation to the target. Such a case is, for example, that in which observation is performed in a system with few noises, such as blood, or the contrast agent according to this Example has a high accumulation to the target.

### Example 16

### (Evaluation of tumor accumulation and HER2 specificity of scFv-NP)

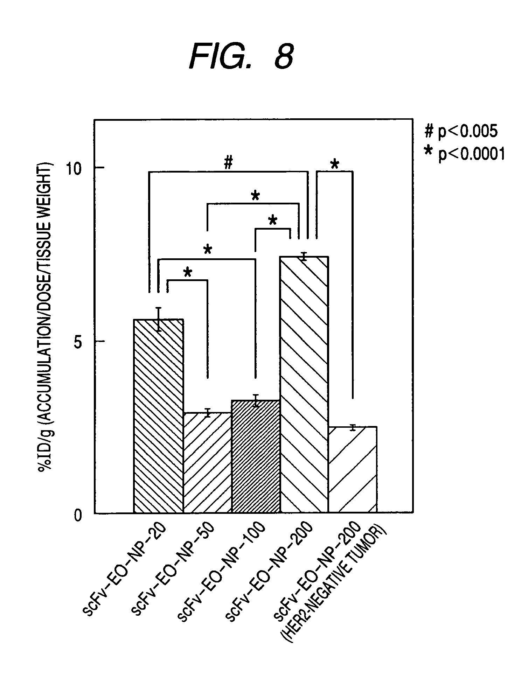

FIG. 8 illustrates the tumor accumulations (at 24 hours after administration; indicated in %ID/g) of scFv-immobilized iron oxide particles with varying particle sizes administered to cancer-bearing mice. This diagram illustrates %ID/g of each of the scFv-EO-NP-20, the scFv-EO-NP-50, the scFv-EO-NP-100, and the scFv-EO-NP-200 in tumor (N87 cells) (results of Example 14) and %ID/g of the scFv-EO-NP-200 in HER2-negative tumor (SUIT2 cells) (tested according to Examples 9 and 14 except that SUIT2 cells were used instead of N87 cells). Results of testing statistically significant difference of each %ID/g are also illustrated (in the diagram, * and # represent significant difference). As a result, the tumor accumulation (HER2-positive tumor) was highest in the scFv-EO-NP-200, followed by the scFv-EO-NP-20 and the scFv-EO-NP-50 and the scFv-EO-NP-100 (no significant difference between scFv-EO-NP-50 and scFv-EO-NP-100). Moreover, it was confirmed that the accumulation of the scFv-EO-NP-200 to HER2-negative tumor (SUIT2 cells) is significantly lower than to HER2-positive tumor (N87 cells). This means that the tumor accumulation of the scFv-EO-NP-200 of this Example is specific for HER2, demonstrating that the scFv-EO-NP-200 can sufficiently function as a contrast agent for photoacoustic imaging that can be used to specifically detect , for example, a HER2 molecule. In the test of the scFv-EO-NP-200, the N87 and SUIT2 tumors had a size of approximately 1 to 3 mm. The influence of the tumor size on tumor accumulation in this system is not clear. However, the tumor size is likely to influence tumor accumulation.

### Example 17

### (Binding between HER2-binding peptide and 200-nm iron oxide particle having ethylene oxide linker)

The particle containing an iron oxide particle used was nanomag (registered trademark)-D (manufactured by Micromod Partikeltechnologie GmbH; average particle size: 200 nm, PEG linker molecular weight: 300), a maleimide-surface modified particle containing a 200-nm iron oxide particle (hereinafter, abbreviated to NP-EO-Maleimide-200). A HER2-binding peptide (amino acid sequence: MARSGLGGKGSC (SEQ ID NO: 3); hereinafter, abbreviated to HBP) was dissolved in a phosphoric acid buffer (2.68 mM KCl/137 mM NaCl/1.47 mM KH2PO4/1 mM Na2HPO4/5 mM EDTA, pH 7.4) and reacted with the NP-EO-Maleimide-200 at 25°C for approximately 2 hours. The reaction was performed at a reaction molar ratio of feed (HBP/particle containing an iron oxide particle) of 1×10⁶. After the reaction, HBP unbound to the particle containing an iron oxide particle was removed by ultrafiltration using Amicon Ultra-4 (Nihon Millipore K.K.) having a pore size of 100 kDa to obtain a complex of the HBP and the particle containing an iron oxide particle. After the ultrafiltration, the amount of the HBP immobilized on the particle containing an iron oxide particle was calculated by quantifying the unreacted HBP contained in the filtrate. The amount of the HBP immobilized on the particle containing an iron oxide particle was 1.8×10⁵ (HBP/particle containing an iron oxide particle). Hereinafter, the HBP-immobilized particle containing an iron oxide particle thus obtained is referred to as HBP1-EO-NP-200. The average hydrodynamic diameter of the HBP1-EO-NP-200 was determined by dynamic light scattering to be 161.1 nm (number-average distribution). Moreover, the particle had a zeta potential of -6.5 mV in PBS. The HBP1-EO-NP-200 had a molar absorption coefficient (ε) of 1.1×10¹⁰ (M-1×cm⁻¹) at 710 nm and had a photoacoustic signal (V/J/nM) of 219.1 at 710 nm.

### Example 18

### (Preparation of 200-nm iron oxide particles with varying numbers of HER2-binding peptides immobilized thereon)

200-nm iron oxide particles with varying numbers of HER2-binding peptides immobilized thereon were prepared according to Example 17. The same procedures as in Example 17 were performed except that each reaction was performed at a reaction molar ratio of feed (HBP/particle containing an iron oxide particle) of 1×10⁵, 2×10⁴, or 2×10³. The obtained HER2-binding peptide-immobilized particles containing an iron oxide particle are abbreviated to HBP2-EO-NP-200, HBP3-EO-NP-200, and HBP4-EO-NP-200, respectively. The amounts of the HBP immobilized on the particles containing an iron oxide particle (HBP/particle containing an iron oxide particle) were 4.4×10⁹, 1.4×10⁴, and 2.0×10³, respectively (2.0×10³ was indicated in the molar amount of the peptide feeded). Their average hydrodynamic diameters and zeta potentials hardly differed from those of the HBP1-EO-NP-200 prepared in Example 17.

### Example 19

### (Evaluation of binding capability of HER2-binding peptide-immobilized particle containing iron oxide particle to HER2-expressing cells)

According to Example 8, a Scatchard plot was prepared from the saturation binding curve of each particle for N87 cells, and an apparent equilibrium dissociation constant (Kd) of the particle for the cell was determined. As a result, the equilibrium dissociation constants (Kd) of the HBP1-EO-NP-200, the HBP2-EO-NP-200, and the HBP3-EO-NP-200 for N87 cells were 0.67 nM, 0.43 nM, and 0.49 nM, respectively. For the HBP4-EO-NP-200 having the smallest number of the HER2-binding peptide immobilized thereon, the equilibrium dissociation constant (Kd) was not obtained from the Scatchard plot due to its weak binding. The specific equilibrium dissociation constant (Kd) of the HER2-binding peptide for HER2 is considered to be 1.0 µM or higher. As shown in this Example, the immobilization of the HER2-binding peptide in large amounts to the particle produced a particle containing an iron oxide particle that is capable of strongly binding to HER2.

### Example 20

### (Preparation of HER2-binding peptide-immobilized iron oxide particle having different molecular weight of ethylene oxide linker)

According to Examples 17 and 18, HBP-EO-NP-200 having an ethylene oxide linker molecular weight of 2000 was prepared. The same procedures as in Examples 17 and 18 were performed except that the particle containing an iron oxide particle used was nanomag (registered trademark)-D (manufactured by Micromod Partikeltechnologie GmbH; average particle size: 200 nm, PEG linker molecular weight: 2000), a maleimide-surface modified particle containing a 200-nm iron oxide particle (hereinafter, abbreviated to NP-EO2k-Maleimide-200). The obtained HER2-binding peptide-immobilized particle containing an iron oxide particles are abbreviated to HBP1-EO2k-NP-200, HBP2-EO2k-NP-200, HBP3-EO2k-NP-200, and HBP4-EO2k-NP-200, respectively. The amounts of the HBP immobilized on the particles containing an iron oxide particle (HBP/particle containing an iron oxide particle) were 2.5×10⁵, 4.9×10⁴, 1.2×10⁴, and 2.0×10³, respectively (2.0×10³ was indicated in the molar amount of the peptide feeded). Their average hydrodynamic diameters (cumulant values) were 248 nm, 207 nm, 250 nm, and 211 nm, respectively. Their zeta potentials were -2.6 mV, -2.8 mV, -2.8 mV, and -3.1 mV, respectively, in PBS.

According to Example 8, a Scatchard plot was prepared from the saturation binding curve of each particle for N87 cells, and an apparent equilibrium dissociation constant (Kd) of the particle for the cell was determined. As a result, the equilibrium dissociation constants (Kd) of the HBP1-EO2k-NP-200, the HBP2-EO2k-NP-200, the HBP3-EO2k-NP-200, and the HBP4-EO2k-NP-200 for N87 cells were 0.07 nM, 0.09 nM, 0.08 nM, and 0.11 nM, respectively.

### Example 21

### (Mouse pharmacokinetics evaluation of HBP-EO-NP-200)

The HBP-EO-NP-200 was evaluated for its pharmacokinetics in cancer-bearing mice. According to Example 9, an RI-labeled particle was prepared, intravenously administered to cancer-bearing mouse models, and examined for its pharmacokinetics by evisceration. The dose of the particle to the mice was 0.22 mg iron/mouse. The number of the particle administered was 3.2×10¹⁰ particles/mouse.

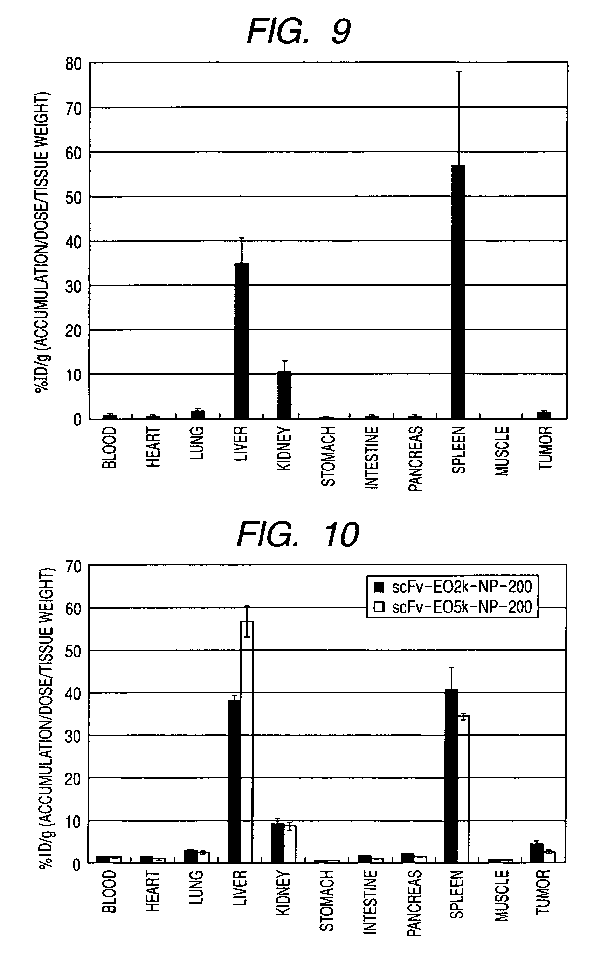

The results of pharmacokinetics at 24 hours after administration are illustrated in FIG. 9. FIG. 9 is a graph illustrating accumulation (value normalized against dose and tissue weight) in each tissue. The tumor (N87 cell) accumulation was 1.5%ID/g.

### Example 22

### (Preparation of artificial antibody-immobilized 200-nm iron oxide particle having varying molecular weights of ethylene oxide linkers)

According to Example 12, an artificial antibody scFv was bound to each particle containing a 200-nm iron oxide particle with an ethylene oxide linker having a molecular weight of 2000 or 5000. The same procedures as in Example 12 were performed except that the particles containing an iron oxide particle used were: nanomag (registered trademark)-D (manufactured by Micromod Partikeltechnologie GmbH; average particle size: 200 nm, PEG linker molecular weight: 2000), a maleimide-surface modified particle containing a 200-nm iron oxide particle (hereinafter, abbreviated to NP-EO2k-Maleimide-200); and nanomag (registered trademark)-D (manufactured by Micromod Partikeltechnologie GmbH; average particle size: 200 nm, PEG linker molecular weight: 5000), another maleimide-surface modified particle containing a 200-nm iron oxide particle (hereinafter, abbreviated to NP-EO5k-Maleimide-200). The scFv-immobilized particles containing an iron oxide particle thus obtained are abbreviated to scFv-EO2k-NP-200 and scFv-EO5k-NP-200, respectively. The average hydrodynamic diameters (number-average distribution) of the scFv-EO2k-NP-200 and the scFv-EO5k-NP-200 were determined by dynamic light scattering to be 175.6 nm and 180.9 nm, respectively. The amounts of the scFv immobilized on the particles containing an iron oxide particle (scFv/particle) were 1490 and 1380, respectively. Moreover, these particles had a zeta potential of -3.9 mV and -4.0 mV, respectively, in PBS.

The scFv-EO2k-NP-200 and the scFv-EO5k-NP-200 had a molar absorption coefficient (ε) of 1.3×10¹⁰ (M⁻¹×cm⁻¹) and 1.1×10¹⁰ (M⁻¹×cm⁻¹), respectively, at 710 nm and had a photoacoustic signal (V/J/nM) of 260.0 and 168.0, respectively, at 710 nm.

According to Example 8, a Scatchard plot was prepared from the saturation binding curve of each particle for N87 cells, and an apparent equilibrium dissociation constant (Kd) of the particle for the cell was determined. As a result, the equilibrium dissociation constants (Kd) of the scfv-E02k-NP-200 and the scFv-EO5k-NP-200 for N87 cells were 0.22 nM and 0.04 nm, respectively.

### Example 23

### (Evaluation of mouse pharmacokinetics of scFv-EO2k-NP-200 and scFv-EO5k-NP-200)

The scFv-EO2k-NP-200 and the scFv-EO5k-NP-200 were evaluated for their pharmacokinetics in cancer-bearing mice. According to Example 9, each RI-labeled particle was prepared, intravenously administered to cancer-bearing mouse models, and examined for its pharmacokinetics by evisceration. The dose of the particle to the mice was 0.22 mg iron/mouse. The number of the particle administered was 3.2×10¹⁰ particles/mouse.

The results of pharmacokinetics at 24 hours after administration are illustrated in FIG. 10. FIG. 10 is a graph illustrating accumulation (value normalized against dose and tissue weight) in each tissue. The tumor (N87 cell) accumulations of the scFv-EO2k-NP-200 and the scFv-EO5k-NP-200 were 4.0%ID/g and 2.2%ID/g, respectively.

### Example 24

### (Photoacoustic imaging using scFv-EO-NP-200 hypodermically administered to mice)

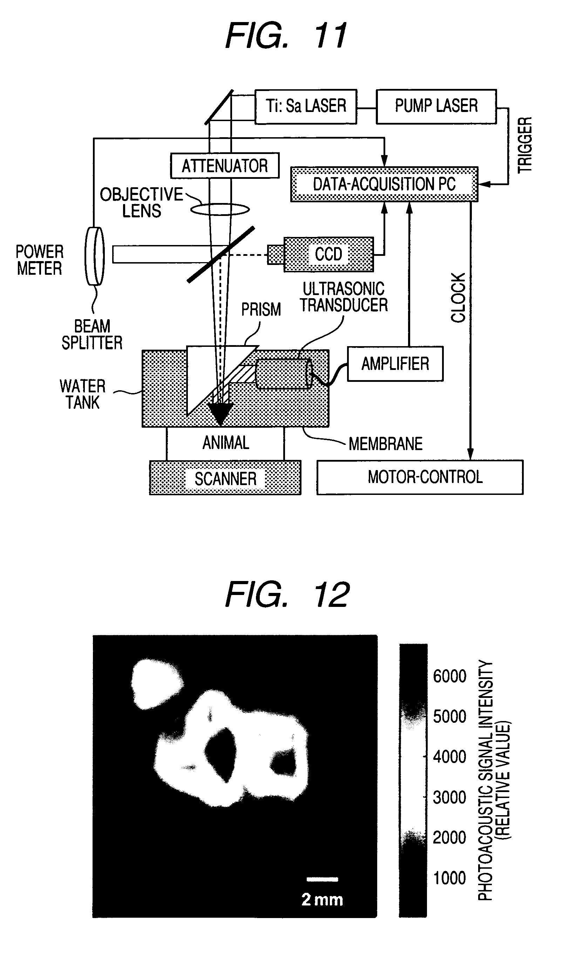

FIG. 11 illustrates a simplified diagram of a manufactured photoacoustic imaging apparatus for small animals. Each small animal was irradiated with laser (wavelength: 710 nm, pulse width: approximately 10 nanoseconds, 10 HZ repetition, beam diameter: approximately 2 mm) from a TI:SA laser source with the laser appropriately attenuated. The formed photoacoustic signal was detected using a ultrasonic transducer (BD=1.5 mm, FC=3.5 MHZ), amplified using an amplifier, and then incorporated to an oscilloscope (50Ω INPUT IMPEDANCE, 2.5GS/S, RECORD LENGTH 10 K). The scanning range was set to 3.5 cm square.

The scFv-EO-NP-200 prepared in Example 12 was used as one example of the contrast agent for photoacoustic imaging. 100 µL of a PBS solution of the scFv-EO-NP-200 (iron concentration: 2.2 mg/mL) was hypodermically injected to each BALB/c Slc-nu/nu mouse (Japan SLC, Inc.). Then, the mouse was fixed to the PA imaging apparatus under anesthesia to perform photoacoustic imaging using the scFv-EO-NP-200 under the skin of the mouse. The results are illustrated in FIG. 12. As is evident from FIG. 12, a strong photoacoustic signal was confirmed from the scFv-EO-NP-200 (iron: 0.2 mg).

### Example 25

### (Molar absorption coefficients and photoacoustic signals against particle size of iron oxide particle)

The molar absorption coefficients and photoacoustic signals of iron oxide particles differing in particle size were determined by the following method:

The iron oxide particles differing in particle size used were a polymer-coated 5-nm iron oxide particle (iron atom concentration: 1.45 mg/ml, iron oxide particle concentration: 10 nmol/ml; SHP-05, manufactured by Ocean NanoTech), a polymer-coated 10-nm iron oxide particle (iron atom concentration: 5 mg/ml, iron oxide particle concentration: 4.3 nmol/ml; SHP-10, manufactured by Ocean NanoTech), a polymer-coated 20-nm iron oxide particle (iron atom concentration: 5 mg/ml, iron oxide particle concentration: 0.55 nmol/ml; SHP-20, manufactured by Ocean NanoTech), and a polymer-coated 50-nm iron oxide particle (iron atom concentration: 5 mg/ml, iron oxide particle concentration: 0.034 nmol/ml; SHP-50, manufactured by Ocean NanoTech). These particle sizes are primary particle sizes, not secondary particle sizes.

Each iron oxide particle was dispersed in water to obtain an iron oxide particle dispersion. The absorbance was measured at 710, 750, 800, and 850 nm using a spectrophotometer (Lambda Bio 40, manufactured by PerkinElmer Inc.). The measurement vessel used was a poly(styrene) cuvette having a width of 1 cm and an optical path length of 0.1 cm. To this vessel, 200 µl of the iron oxide particle dispersion was added and measured for its absorbance. The iron atom concentrations and particle concentrations of the iron oxide particles were determined from the document provided by Ocean NanoTech.

The molar absorption coefficients per mol of iron atoms and the molar absorption coefficients per mol of iron oxide particles were calculated from the absorbances, the iron atom concentrations, and the particle concentrations.

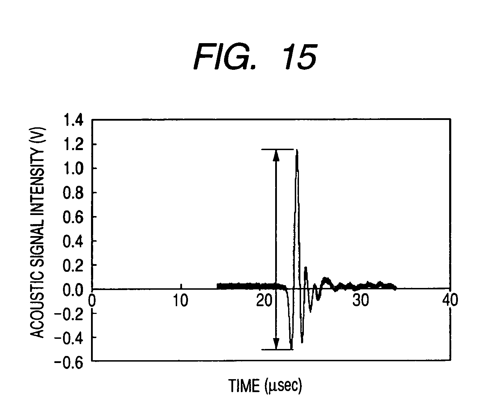

In photoacoustic signal measurement, each contrast agent for photoacoustic imaging dispersed in water was irradiated with pulse laser light. Photoacoustic signals from the contrast agent were detected using a piezoelectric element and amplified using a high-speed preamplifier, and their waveforms were then obtained using a digital oscilloscope. Specific conditions are as follows: the pulse laser source used was a titanium-sapphire laser (LT-2211-PC, manufactured by LOTIS LTD.). The conditions involved a wavelength of 710, 750, 800, and 850 nm, an energy density of approximately 20 to 50 mJ/cm² (depending on the selected wavelength), a pulse width of approximately 20 nanoseconds, and a pulse repetition frequency of 10 Hz. The measurement vessel used for containing the contrast agent for photoacoustic imaging dispersed in water was the poly(styrene) cuvette described above. The piezoelectric element used for detecting photoacoustic signals was a non-convergence-type ultrasonic transducer (V303, manufactured by Panametrics-NDT) having an element diameter of 1.27 cm and a central frequency of 1 MHz. The measurement vessel and the piezoelectric element were dipped at a distance of 2.5 cm therebetween in a glass container filled with water. The high-speed preamplifier used for amplifying photoacoustic signals was an ultrasonic preamplifier (Model 5682, manufactured by OLYMPUS CORP.) having an amplification degree of +30 dB. The amplified signals were input to a digital oscilloscope (DPO4104, manufactured by Tektronix). The poly(styrene) cuvette was irradiated with pulse laser light from outside of the glass container. A portion of light scattered therefrom was detected using a photodiode and input as a trigger signal to the digital oscilloscope. The digital oscilloscope was set to a 32 run-averaging display mode to obtain an average photoacoustic signal of 32 laser pulse irradiations. FIG. 15 illustrates the waveform of the typical photoacoustic signal. As indicated in the arrow in the diagram, photoacoustic signal intensity (V) was determined from the waveform. The obtained photoacoustic signal intensity was divided by the energy (J) of the irradiated pulse laser, and the obtained value is defined as a photoacoustic signal (VJ⁻¹) for normalization and was used below as an evaluation unit.

The photoacoustic signal for normalization per mol of iron atoms and the photoacoustic signal for normalization per mol of iron oxide particles were calculated from the iron atom concentration and the particle concentration, respectively.

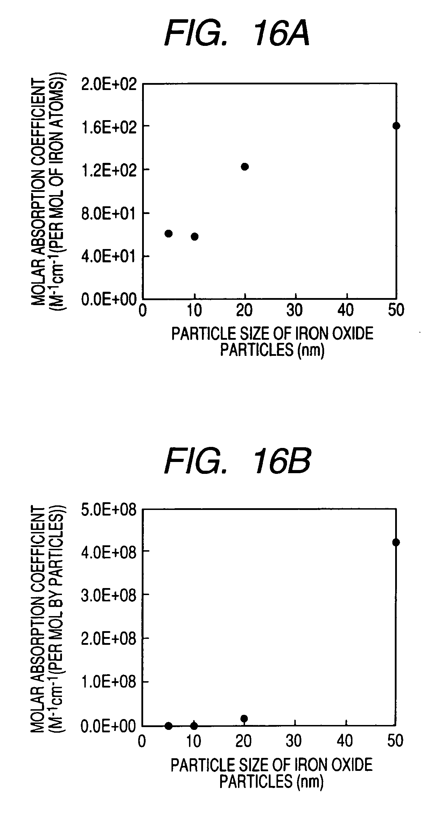

FIGS. 16A to 16D illustrate the molar absorption coefficients and photoacoustic signals of the iron oxide particles differing in particle size. FIG. 16A illustrates the molar absorption coefficient per mol of iron atoms contained in each iron oxide particle at 710 nm. FIG. 16B illustrates the molar absorption coefficient per mole of iron oxide particles at 710 nm. The molar absorption coefficient per mol of iron atoms exhibited an increasing tendency with increases in the particle size of the iron oxide particle. Such increases in molar absorption coefficient per mol of iron atoms were also observed at other wavelengths. Owing to this effect, the molar absorption coefficient per mol of iron oxide particles was significantly increased with increases in the particle size of the iron oxide particle.

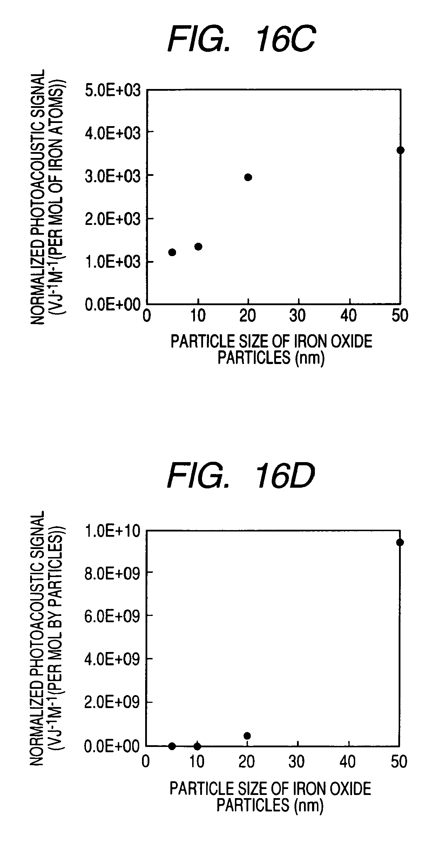

FIG. 16C illustrates the photoacoustic signal for normalization per mol of iron atoms contained in each iron oxide particle at 710 nm. FIG. 16D illustrates the photoacoustic signal for normalization per mol of iron oxide particles at 710 nm. The photoacoustic signal for normalization per mol of iron atoms was increased with increases in the particle size of the iron oxide particle. Likewise, such increases in photoacoustic signal for normalization per mol of iron atoms were also observed at other wavelengths. Owing to this effect, the photoacoustic signal for normalization per mol of iron oxide particles was significantly increased with increases in the particle size of the iron oxide particle.

The iron oxide particle having a larger particle size absorbed light in larger amounts and thereby emitted a stronger photoacoustic signal. The increases in the amount of light absorbed and in photoacoustic signal were observed, particularly in the 15-nm or larger iron oxide particles.

### Example 26

### (Preparation of contrast agent 1 (NP1) for photoacoustic imaging)

A contrast agent for photoacoustic imaging including polyoxyethylene sorbitan fatty acid ester, a hydrophobic polymer, and an iron oxide particle was prepared by the following method:

An oleic acid-coated 50-nm iron oxide particle (13.8 mg, approximately 50 nm; SOR-50, manufactured by Ocean NanoTech) and a poly(lactic acid-*co*-glycolic acid) (9.2 mg, M.W. 20000, lactic acid:glycolic acid=1:1; PLGA5020, manufactured by Wako Pure Chemical Industries, Ltd.) were added to chloroform (1 ml). The mixture was irradiated in an ultrasonic bath for 10 minutes to prepare a chloroform solution.

Next, the chloroform solution was added to an aqueous solution (12 ml) containing Tween 20 (60 mg; manufactured by Kishida Chemical Co., Ltd.) dissolved therein to prepare a mixed solution.

Then, the mixed solution was treated with an ultrasonic disruptor (UD-200, manufactured by TOMY SEIKO CO., LTD.) for 4 minutes to prepare an O/W-type emulsion.

Next, chloroform was evaporated from the dispersoid of the emulsion under reduced pressure of 100 hPa at 40°C for 1 hour or longer to obtain a water dispersion of a contrast agent 1 for photoacoustic imaging. The contrast agent 1 for photoacoustic imaging was purified by centrifugation. Hereinafter, this obtained contrast agent 1 for photoacoustic imaging is also abbreviated to NP1.

### Example 27

### (Preparation of contrast agent 2 (NP2) for photoacoustic imaging)

A contrast agent for photoacoustic imaging including phospholipid, polyoxyethylene sorbitan fatty acid ester, a hydrophobic polymer, and an iron oxide particle was prepared by the following method:

The oleic acid-coated 50-nm iron oxide particle (13.8 mg; SOR-50, manufactured by Ocean NanoTech) and the poly(lactic acid-*co*-glycolic acid) (9.2 mg) were added to chloroform (1 ml). The mixture was irradiated in an ultrasonic bath for 10 minutes to prepare a chloroform solution.

Next, the chloroform solution was added to an aqueous solution (12 ml) containing Tween 20 (60 mg) and phospholipid (7.3 mg; DSPE-PEG-MAL, (N-[(3-maleimide-1-oxopropyl)aminopropyl polyethyleneglycol-carbonyl] distearoylphosphatidyl-ethanolamine), manufactured by NOF CORP.) dissolved therein to prepare a mixed solution.

Then, the mixed solution was treated with an ultrasonic disruptor for 4 minutes to prepare an O/W-type emulsion.

Next, chloroform was evaporated from the dispersoid of the emulsion under reduced pressure of 100 hPa at 40°C for 1 hour or longer to obtain a water dispersion of a contrast agent 2 for photoacoustic imaging. The contrast agent 2 for photoacoustic imaging was purified by centrifugation. Hereinafter, this obtained contrast agent 2 for photoacoustic imaging is also abbreviated to NP2.

### (Physical property evaluation of NP1 and NP2)

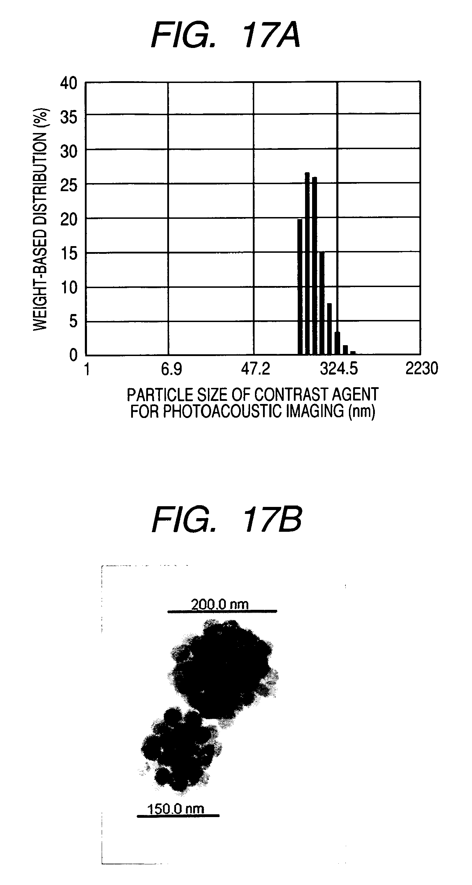

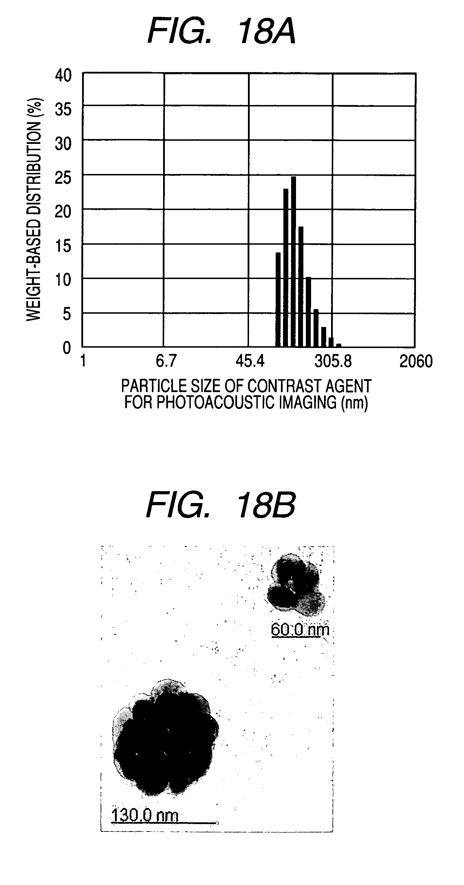

The particle sizes of the NP1 and the NP2 were determined using DLS. The NP1 and the NP2 were separately dispersed in water and measured for their average particle sizes using a dynamic light scattering analyzer (DLS; ELS-Z, manufactured by OTSUKA ELECTRONICS CO., LTD.). The results are illustrated in FIGS. 17A and 18A. The average particle sizes (weight-average) of the NP1 and the NP2 were 205 and 176 nm, respectively.

The NP1 and the NP2 were observed using a transmission electron microscope (TEM; H800, manufactured by Hitachi, Ltd.). FIGS. 17B and 18B are respectively a TEM photograph of the contrast agent for photoacoustic imaging. The contrast agent for photoacoustic imaging containing a plurality of iron oxide particles could be observed.

The amounts of iron oxide particles contained in the NP1 and the NP2 were determined using an emission spectrometer (ICP; CIROS CCD, manufactured by SPECTRO Analytical Instruments GmbH). Each contrast agent for photoacoustic imaging was dissolved in concentrated nitric acid. The amount of Fe was determined using ICP, and the weight of the iron oxide particle was calculated. A water dispersion of the contrast agent for photoacoustic imaging was lyophilized and then measured for its weight to determine the weight of the whole contrast agent for photoacoustic imaging. From the weight of the iron oxide particle and the weight of the contrast agent for photoacoustic imaging, the iron oxide particle content in the contrast agent for photoacoustic imaging was calculated to be 58 and 63 (wt)% for NP1 and NP2, respectively. The contrast agents for photoacoustic imaging having a high iron oxide particle content could be obtained.

The obtained NP1 and NP2 were evaluated for their light absorption properties by determining molar absorption coefficients per mol of particles of the contrast agents for photoacoustic imaging. The light absorptions of the NP1 and the NP2 dispersed in water were measured using a spectrophotometer. The particle concentrations of the contrast agents for photoacoustic imaging were calculated from the values of the physical properties obtained by these methods. The molar absorption coefficients per mol of particles of the contrast agents for photoacoustic imaging at wavelengths of 710, 750, 800, and 850 nm are summarized in Table 4 below. The molar absorption coefficients of the NP1, the NP2, and Resovist (registered trademark) at the wavelength of 710 nm were 2.1×10¹⁰, 9.9×10⁹, and 1.6×10⁶ (M⁻¹cm⁻¹ (per mol of particles)), respectively. The contrast agents for photoacoustic imaging having more excellent light absorption than that of Resovist (registered trademark) as a known contrast agent for photoacoustic imaging were obtained.

**Table 4**

| Wavelength (nm) | Molar absorption coefficient | |
|---|---|---|
| | (M⁻¹cm⁻¹ (per mol of particles)) | |
| | NP1 | NP2 |
| 710 | 2.1×10¹⁰ | 9.9×10⁹ |
| 750 | 1.8×10¹⁰ | 8.6×10⁹ |
| 800 | 1.6×10¹⁰ | 7.7×10⁹ |
| 850 | 1.5×10¹⁰ | 7.2×10⁹ |

Table 4. Molar absorption coefficient per mol of particles of contrast agent for photoacoustic imaging at each wavelength

Table 5 illustrates the photoacoustic signals for normalization per particle of the NP1 and the NP2. The photoacoustic signals for normalization per mol of particles of the NP1, the NP2, and Resovist (registered trademark) at a wavelength of 710 nm were 3.5×10¹¹, 2.0×10¹¹, and 6.0×10⁷ (VJ⁻¹M⁻¹ (per mol of particles)), respectively. The contrast agents for photoacoustic imaging emitting a stronger acoustic signal than that of Resovist (registered trademark) as a known contrast agent for photoacoustic imaging were obtained.

**[Table 5]**

| Wavelength (nm) | Photoacoustic signal for normalization | |
|---|---|---|
| | (VJ⁻¹M⁻¹ (per mol of particles)) | |
| | NP1 | NP2 |
| 710 | 3.5×10¹¹ | 2.0×10¹¹ |
| 750 | 3.0×10¹¹ | 1.9×10¹¹ |
| 800 | 2.7×10¹¹ | 2.0×10¹¹ |
| 850 | 3.1×10¹¹ | 1.8×10¹¹ |

Table 5. Photoacoustic signal for normalization per mol of particles of contrast agent for photoacoustic imaging at each wavelength

### (Quantification of phospholipid on NP2)

The amount of the phospholipid introduced to the NP2 surface was determined using the activity of the terminal maleimide group. The NP2 and L-cysteine were mixed and incubated overnight to react the maleimide group on the NP2 with a thiol group on the L-cysteine through covalent binding. The NP2 was precipitated by centrifugation, and the supernatant was collected to collect unreacted L-cysteine. The unreacted L-cysteine was mixed with 5,5'-dithiobis(2-nitrobenzoic acid) and quantified by measuring the absorbance at 420 nm. The amount of the phospholipid on one NP2 particle was 5.6×10⁵ molecules/particle.

### Example 28

### (Preparation of single-chain antibody hu4D5-8scFv)

Based on the gene sequence (hu4D5-8) of a HER2-binding IgG variable region, a gene hu4D5-8scFv encoding a single-chain antibody (scFv) was prepared. First, cDNA including the VL and VH genes of hu4D5-8 linked via cDNA encoding a peptide (GGGGS)₃ was prepared. NcoI- and NotI restriction sites were introduced to the 5' and 3' ends thereof, respectively. The nucleotide sequence is shown below. 
5'-
CCATGGATATCCAGATGACCCAGTCCCCGAGCTCCCTGTCCGCCTCTGTGGGCGATAGG
GTCACCATCACCTGCCGTGCCAGTCAGGATGTGAATACTGCTGTAGCCTGGTATCAACA
GAAACCAGGAAAAGCTCCGAAACTACTGATTTACTCGGCATCCTTCCTCTACTCTGGAG
TCCCTTCTCGCTTCTCTGGATCCAGATCTGGGACGGATTTCACTCTGACCATCAGCAGT
CTGCAGCCGGAAGACTTCGCAACTTATTACTGTCAGCAACATTATACTACTCCTCCCAC
GTTCGGACAGGGTACCAAGGTGGAGATCAAAGGCGGTGGTGGCAGCGGTGGCGGTGGCA
GCGGCGGTGGCGGTAGCGAGGTTCAGCTGGTGGAGTCTGGCGGTGGCCTGGTGCAGCCA
 
GGGGGCTCACTCCGTTTGTCCTGTGCAGCTTCTGGCTTCAACATTAAAGACACCTATAT
ACACTGGGTGCGTCAGGCCCCGGGTAAGGGCCTGGAATGGGTTGCAAGGATTTATCCTA
CGAATGGTTATACTAGATATGCCGATAGCGTCAAGGGCCGTTTCACTATAAGCGCAGAC
ACATCCAAAAACACAGCCTACCTGCAGATGAACAGCCTGCGTGCTGAGGACACTGCCGT
CTATTATTGTTCTAGATGGGGAGGGGACGGCTTCTATGCTATGGACTACTGGGGTCAAG
GAACCCTGGTCACCGTCTCCTCGGCGGCCGC-3′ (SEQ ID NO: 1)
 (Restriction sites are underlined.)

The gene fragment hu4D5-8scFv was inserted downstream of a T7/lac promoter of a plasmid pET-22b (+) (Novagen, Inc.). Specifically, the cDNA was ligated with pET-22b (+) (Novagen, Inc.) digested with restriction enzymes NcoI- and NotI.

*Escherichia coli* BL21(DE3) was transformed with this expression plasmid to obtain a bacterial strain for expression. The obtained bacterial strain was precultured overnight in 4 ml of LB-Amp medium. Then, the whole amount thereof was added to 250 ml of 2xYT medium and shake-cultured at 120 rpm at 28°C for 8 hours. Then, after addition of IPTG (isopropyl-β-D(-)-thiogalactopyranoside) at a final concentration of 1 mM, the bacterial strain was cultured overnight at 28°C. The cultured *Escherichia coli* was centrifuged at 8000×g at 4°C for 30 minutes, and the supernatant of the culture solution was collected. To the obtained culture solution, 60% by weight of ammonium sulfate was added, and proteins were precipitated by salting-out. The solution subjected to salting-out was left standing overnight at 4°C and then centrifuged at 8000×g at 4°C for 30 minutes to collect precipitates. The obtained precipitates were dissolved in 20 mM Tris·HCl/500 mM NaCl buffer and dialyzed against 1 1 of the same buffer. The protein solution thus dialyzed was added to a column charged with His·Bind (registered trademark) Resin (Novagen, Inc.) and purified by metal chelate affinity chromatography via Ni ions. The purified hu4D5-8scFv was confirmed in reducing SDS-PAGE to exhibit a single band and have a molecular weight of approximately 28 kDa. The amino acid sequence of the prepared antibody is shown below. Hereinafter, the hu4D5-8scFv is also abbreviated to scFv.

DIQMTQSPSSLSASVGDRVTITCRASQDVNTAVAWYQQKPGKAPKLLIYSASFLYS
GVPSRFSGSRSGTDFTLTISSLQPEDFATYYCQQHYTTPPTFGQGTKVEIKGGGGS
GGGGSGGGGSEVQLVESGGGLVQPGGSLRLSCAASGFNIKDTYIHWVRQAPGKGLE
WVARIYPTNGYTRYADSVKGRFTISADTSKNTAYLQMNSLRAEDTAVYYCSRWGGD
GFYAMDYWGQGTLVTVSSAAALEHHHHHHGGC
(SEQ ID NO: 2)

### (Binding between single-chain antibody hu4D5-8scFv and NP2)

The buffer of the preparation of the above "preparation of single-chain antibody hu4D5-8sdCv" was replaced by a phosphoric acid buffer containing 5 mM EDTA (2.68 mM KCl/137 mM NaCl/1.47 mM KH₂PO₄/1 mM Na₂HPO₄/5 mM EDTA, pH 7.4). Then, the scFv was reduced at 25°C for approximately 2 hours using a 20-fold molar amount of tris(2-carboxyethyl)phosphine (TCEP). This scFv thus reduced was reacted at 25°C for 3 hours with the NP2 prepared above. The scFv was used in the reaction in an amount 2000 times the molar amount of the NP2 particle. Precipitates of scFv-immobilized NP2 and a supernatant of unreacted scFv were obtained by centrifugation. The unreacted scFv was quantified by liquid chromatography. The scFv immobilized on one NP2 particle was 1.4×10³ molecules/particle.

### Example 29

### (Preparation of contrast agent 3 (NP3) for photoacoustic imaging)

A contrast agent for photoacoustic imaging including an amphiphilic polymer and an iron oxide particle was prepared by the following method:

A poly(maleic anhydride-*co*-styrene) (12.5 mg, average molecular weight: 9500, styrene:maleic anhydride=75:25 (molar ratio); manufactured by Polysciences, Inc.) was dissolved in chloroform (3 ml). This solution was reacted by the addition of MeO-PEG-NH2 (45 mg, average molecular weight: 5000; SUNBRIGHT ME-50EA, manufactured by NOF CORP.) to obtain an amphiphilic polymer (poly(maleic anhydride-*co-*styrene) having a PEG chain introduced therein).

An oleic acid-coated 50-nm iron oxide particle (14 mg; SOR-50, manufactured by Ocean NanoTech) and the amphiphilic polymer (50 mg) were added to chloroform (3 ml). Then, chloroform was evaporated by evaporation.

Then, water (18 ml) was added to the residue. The mixture was treated in an ultrasonic batch for 1 minute to obtain a water dispersion of a contrast agent 3 for photoacoustic imaging. The contrast agent 3 for photoacoustic imaging was purified by centrifugation. Hereinafter, this obtained contrast agent 3 for photoacoustic imaging is also abbreviated to NP3.

The NP3 had an average particle size of 211 nm (weight-average). Moreover, the NP3 was confirmed to have a molar absorption coefficient of 1.9×10¹⁰ (M⁻¹cm⁻¹ (per mol of particles)) per mol of particles and a photoacoustic signal for normalization of 4.8×10¹¹ (V J⁻¹M⁻¹ (per mol of particles)) per mol of particles.

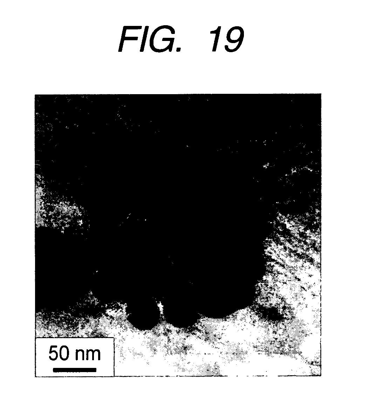

The NP3 was observed using cryo-TEM. The results are illustrated in FIG. 19. From FIG.19, the NP3 was confirmed to contain a plurality of iron oxide particles.

### Example 30

### (Particle size and photoacoustic signal of contrast agent for photoacoustic imaging)

Contrast agents for photoacoustic imaging differing in particle size were prepared by a method shown below. The iron oxide particles used were an oleic acid-coated 50-nm iron oxide particle (SOR-50, manufactured by Ocean NanoTech) and an oleic acid-coated 5-nm iron oxide particle (SOR-05, manufactured by Ocean NanoTech).

The 5-nm or 50-nm iron oxide particle (13.8 mg) and a poly (lactic acid-*co*-glycolic acid) (9.2 mg) were added to chloroform (1 ml). The mixture was irradiated in an ultrasonic bath for 10 minutes to prepare a chloroform solution. Next, various kinds of aqueous solutions (12 ml) containing Tween 20 (60 mg; manufactured by Kishida Chemical Co., Ltd.) dissolved therein were prepared with or without phospholipids of varying types. The chloroform solution was added to each aqueous solution of Tween 20 to prepare a mixed solution. Then, the same procedures as in the NP1 and NP2 preparation methods were performed. The contrast agent for photoacoustic imaging containing the 5-nm iron oxide particle and the contrast agent for photoacoustic imaging containing the 50-nm iron oxide particle were prepared at the same iron atom weights. From TEM observation and iron oxide particle contents in the contrast agents for photoacoustic imaging, it could be confirmed that the obtained contrast agents for photoacoustic imaging densely contained a plurality of 5-nm or 50-nm iron oxide particles.

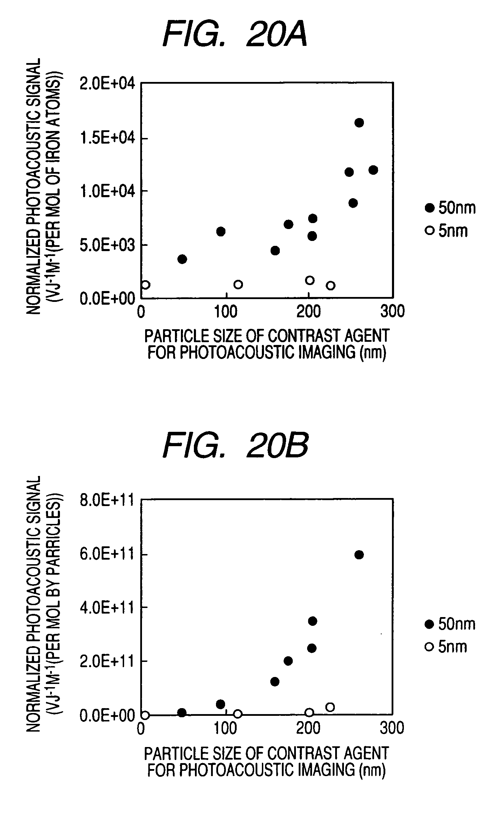

FIGS. 20A and 20B illustrate the relationship between the average particle size (weight-average) of the contrast agent for photoacoustic imaging containing the 50-nm or 5-nm iron oxide particle and photoacoustic signals. Photoacoustic signals for normalization per mol of iron atoms and photoacoustic signals for normalization per mol of particles of each contrast agent for photoacoustic imaging are illustrated as photoacoustic signals in FIGS. 20A and 20B, respectively. The contrast agent for photoacoustic imaging containing the 50-nm iron oxide particle was confirmed to have increases in photoacoustic signal per mol of iron atoms with increases in the particle size of the contrast agent for photoacoustic imaging. This tendency was irrelevant to the preparation method, i.e., the components other than the iron oxide particle. Moreover, owing to this tendency, in the contrast agent for photoacoustic imaging containing the 50-nm iron oxide particle, the photoacoustic signals per mol of particles of the contrast agent for photoacoustic imaging was also increased. Thus, the contrast agent for photoacoustic imaging emitting strong photoacoustic signals was obtained.

### Reference Signs List

- 1: contrast agent for photoacoustic imaging
- 2: iron oxide particle
- 3: amphiphilic compound
- 4: phospholipid
- 5: polyoxyethylene sorbitan fatty acid ester
- 6: amphiphilic polymer
- 7: hydrophobic polymer
- 8: ligand molecule
- 9: contrast agent for photoacoustic imaging
- 10: first liquid
- 11: second liquid
- 12: emulsion
- 13: water

This application claims the benefit of Japanese Patent Applications No. 2009-231994, filed October 5, 2009, and No. 2010-144322, filed June 24, 2010 which are hereby incorporated by reference herein in their entirety.

## Claims

1. A contrast agent for photoacoustic imaging represented by Formula 1:
MNP-((L)1-(P)m)n (Formula 1)
(wherein MNP represents a particle containing an iron oxide particle; L represents a linker molecule; P represents a ligand molecule; 1 represents 0 or 1; and m and n represent an integer of 1 or larger),
the contrast agent for photoacoustic imaging comprising: a particle containing an iron oxide particle that absorbs light in a near-infrared region; and at least one or more ligand molecule(s) immobilized on the particle containing an iron oxide particle, wherein the immobilization density of the ligand molecule is equal to or higher than the cell surface density of a target molecule.

2. The contrast agent for photoacoustic imaging according to claim 1, wherein the number of ligand molecules is 3 or more per the particle containing an iron oxide particle.

3. The contrast agent for photoacoustic imaging according to claim 1 or 2, wherein the ligand molecule is selected from the group consisting of at least a monoclonal antibody, Fab, Fab', F(ab'), a single-chain antibody (scFv), a peptide containing an antibody complementarity determining region (CDR), and an antibody or a peptide that binds to epidermal growth factor receptor 2 (HER2).

4. The contrast agent for photoacoustic imaging according to any one of claims 1 to 3, wherein the linker molecule is polyethylene oxide.

5. The contrast agent for photoacoustic imaging according to claim 4, wherein the polyethylene oxide has a molecular weight from 100 to 10000.

6. The contrast agent for photoacoustic imaging according to any one of claims 1 to 5, wherein the particle containing an iron oxide particle contains dextran as a matrix.

7. The contrast agent for photoacoustic imaging according to any one of claims 1 to 6, wherein the particle containing an iron oxide particle is magnetite (Fe3O4).

8. A photoacoustic imaging method for a target molecule, which is an imaging method for detecting a target molecule using a contrast agent for photoacoustic imaging according to any one of claims 1 to 7, the method comprising at least: administering the contrast agent for photoacoustic imaging to a cell, tissue, animal or human sample; irradiating the sample with pulsed light; and measuring a photoacoustic signal derived from the contrast agent for photoacoustic imaging bound to the target molecule present in the sample.

9. The contrast agent for photoacoustic imaging according to any one of claims 1 to 7, wherein the particle containing an iron oxide particle has a molar absorption coefficient of particles of 109 (M-1×cm-1) or higher.

10. The contrast agent for photoacoustic imaging according to any one of claims 1 to 7, wherein the particle containing an iron oxide particle contains 107 or more iron atoms.

11. A contrast agent for photoacoustic imaging having an iron oxide particle, wherein a single-chain antibody is bound to the particle.

12. A photoacoustic imaging method comprising:
irradiating, with light in a wavelength region of 600 nm to 1300 nm, a sample that has received a contrast agent for photoacoustic imaging according to claim 11; and
detecting an acoustic wave generated from the contrast agent present in the sample.

13. A contrast agent for photoacoustic imaging having an iron oxide particle, wherein the iron oxide particle has a particle size between 15 nm and 500 nm inclusive.

14. A contrast agent for photoacoustic imaging having an iron oxide particle, wherein the iron oxide particle has a particle size between 20 nm and 500 nm inclusive.

15. The contrast agent for photoacoustic imaging according to claim 13 or 14, wherein the iron oxide particle is coated with a hydrophobic polymer selected from the group consisting of a homopolymer comprising a monomer having hydroxycarboxylic acid having 6 or less carbon atoms, or a copolymer comprising two kinds of monomers having the hydroxycarboxylic acid, poly(styrene), and poly(methyl methacrylate).

16. The contrast agent for photoacoustic imaging according to any one of claims 13 to 15, wherein the contrast agent for photoacoustic imaging according to any one of claims 13 to 15 further has an amphiphilic compound, the amphiphilic compound being one or more amphiphilic compound(s) selected singly or in combination from phospholipid, polyoxyethylene sorbitan fatty acid esters, or amphiphilic polymers.

17. The contrast agent for photoacoustic imaging according to claim 16, wherein the polyoxyethylene sorbitan fatty acid ester is selected from the group consisting of Tween 20, Tween 40, Tween 60, and Tween 80.

18. The contrast agent for photoacoustic imaging according to claim 16, wherein the phospholipid is a phosphatidyl phospholipid having one or more functional group(s) selected from the group consisting of amino, carboxyl, NHS (N-hydroxysuccinimide), maleimide, methoxy, and hydroxy groups, and having a PEG (polyethylene glycol) chain.

19. The contrast agent for photoacoustic imaging according to claim 16, wherein the amphiphilic polymer is selected from the group consisting of a poly(maleic anhydride-alt-octadecen) having a PEG chain introduced therein, a poly(maleic anhydride-co-styrene) having a PEG chain introduced therein, poly(lactic acid) having a PEG chain introduced therein, poly(lactic acid-co-glycolic acid) having a PEG chain introduced therein, poly(ethylene glycol-co-propyleneoxide) and poly(vinyl alcohol).

20. The contrast agent for photoacoustic imaging according to any one of claims 13 to 19, wherein a ligand molecule is bound to at least a portion of the amphiphilic compound.

21. The contrast agent for photoacoustic imaging according to claim 20, wherein the ligand molecule is selected from the group consisting of an antibody, an antibody fragment, an enzyme, a bioactive peptide, a glycopeptide, a sugar chain, a lipid, and a molecular recognition compound.

22. A photoacoustic imaging method comprising:
irradiating, with light in a wavelength region of 600 nm to 1300 nm, a sample that has received a contrast agent for photoacoustic imaging according to any of claims 13 to 20; and
detecting an acoustic wave generated from the contrast agent present in the sample.
